(19) Europäisches Patentamt
European Patent Office
Office européen des brevets

(11) EP 4 555 919 A1

(12) EUROPEAN PATENT APPLICATION
published in accordance with Art. 153(4) EPC

(43) Date of publication:
21.05.2025 Bulletin 2025/21

(21) Application number: 23868017.7

(22) Date of filing: 31.08.2023

(51) International Patent Classification (IPC):
A61B 3/12 $^{(2006.01)}$   A61B 3/14 $^{(2006.01)}$

(52) Cooperative Patent Classification (CPC):
A61B 3/12; A61B 3/14

(86) International application number:
PCT/JP2023/031792

(87) International publication number:
WO 2024/062893 (28.03.2024 Gazette 2024/13)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 22.09.2022 JP 2022151557

(71) Applicant: Topcon Corporation
Tokyo 174-8580 (JP)

(72) Inventors:
• SUZUKI Masaya
  Tokyo 174-8580 (JP)
• FUJINO Makoto
  Tokyo 174-8580 (JP)
• SAKAI Jun
  Tokyo 174-8580 (JP)

(74) Representative: Gagel, Roland
Patentanwalt Dr. Roland Gagel
Landsberger Strasse 480a
81241 München (DE)

(54) **FUNDUS IMAGING DEVICE, METHOD FOR CONTROLLING FUNDUS IMAGING DEVICE, AND PROGRAM**

(57) This fundus imaging device includes two or more curved mirrors, an illumination optical system, an image sensor, and a control unit. The illumination optical system irradiates the fundus of an eye under examination with slit-shaped illumination light via the two or more curved mirrors, and illuminates the fundus while moving the illuminated region. The image sensor can be disposed at a fundus-conjugate position, which is substantially optically conjugate with the fundus, and receives return light from the eye under examination. The control unit controls at least the illumination optical system. The longitudinal direction of an image formed by the illumination light on the fundus is substantially parallel to a plane that includes two or more focal points of the two or more curved mirrors. The control unit controls the illumination optical system so that the abovementioned image moves in a direction intersecting the longitudinal direction on the fundus.

FIG. 2

EP 4 555 919 A1

**Description**

[TECHNICAL FIELD]

This invention relates to a fundus imaging apparatus, a method of controlling a fundus imaging apparatus, and a program.

[BACKGROUND ART]

Fundus imaging apparatuses (fundus photography apparatuses) used for screening or treatment of eye diseases are expected to be capable of easily imaging (observing) the fundus of the eye to be examined with a wide field of view. Specifically, the fundus imaging apparatuses capable of imaging the fundus of the eye to be examined at a wide imaging (shooting) angle of view of more than 80 degrees in a single imaging (shot) are expected. As such fundus imaging apparatuses, scanning laser ophthalmoscopes (SLOs) are known. SLO is an apparatus configured to form an image of the fundus by scanning the fundus with light to detect returning light of the light with a light receiving device.
For example, Patent Document 1 discloses a scanning ophthalmoscope that can scan a retina at a wide angle by moving a two-dimensional collimated scan using a polygon mirror and a plane mirror on the eye to be examined using a scan transfer means.
For example, Patent Document 2 and Patent Document 3 disclose a fundus imaging apparatus that can acquire high-contrast images with a simple configuration, by combining a fundus scanning using slit-shaped illumination light and a rolling shutter. In particular, Patent Document 3 discloses a method of acquiring wide-angle fundus images of the eye to be examined by scanning the fundus of the eye to be examined with the slit-shaped illumination light through two ellipsoidal concave mirrors.

[PRIOR ART DOCUMENTS]

[PATENT DOCUMENTS]

[PATENT DOCUMENT 1] Japanese Unexamined Patent Application Publication (Translation of PCT Application) No. 2009-543585
[PATENT DOCUMENT 2] US Patent No. 7831106
[PATENT DOCUMENT 3] WO 2022/124170

[SUMMARY OF THE INVENTION]

[PROBLEMS TO BE SOLVED BY THE INVENTION]

The method disclosed in Patent Document 1 requires a high speed scanner such as a polygon mirror. However, high speed scanners capable of scanning at wide angles are very expensive.
Further, in case of imaging the fundus at a wide angle, an optical path of an illumination light and an optical path of returning light of the illumination light are desirable to be spatially divided at a branch point at a position conjugate to a pupil of the eye to be examined. The method disclosed in Patent Document 2 has difficulty in pupil dividing. In addition, it is extremely difficult to limit the light flux illuminating a wide range on the fundus and the light flux of the returning light within an effective diameter of the optical system while maintaining the working distance.
Furthermore, the method disclosed in Patent Document 3 makes it possible to photograph the fundus of the eye to be examined at a wide angle at low cost and with a simple configuration. However, due to the susceptibility to aberrations of the ellipsoidal mirror, advanced optical design becomes necessary.
These problems are common not only when acquiring wide-angle images of the fundus, but also when acquiring images of the fundus.
The present invention has been made in view of such circumstances, and one of objects of the present invention is to provide a new technique for acquiring higher-quality images of a fundus of an eye to be examined at low cost and with a simple configuration, without requiring advanced optical design.

[MEANS OF SOLVING THE PROBLEMS]

One aspect of embodiments is a fundus imaging apparatus, including: two or more curved mirrors; an illumination optical system configured to irradiate slit-shaped illumination light onto a fundus of an eye to be examined through the two or more curved mirrors, and to illuminate the fundus while moving an illumination region; an image sensor that is arrangeable at a fundus conjugate position substantially conjugate optically to the fundus, and configured to receive returning light from the eye to be examined; and a controller configured to control at least the illumination optical system, wherein a longitudinal direction of an image formed by the illumination light on the fundus is approximately parallel to a plane including two or more focal points of the two or more curved mirrors, and the controller is configured to control the illumination optical system so that the image moves in a direction intersecting the longitudinal direction on the fundus.

Another aspect of the embodiments is a method of controlling a fundus imaging apparatus including: two or more curved mirrors; an illumination optical system configured to irradiate slit-shaped illumination light onto a fundus of an eye to be examined through the two or more curved mirrors, and to illuminate the fundus while moving an illumination region; and an image sensor that is arrangeable at a fundus conjugate position substantially conjugate optically to the fundus, and configured to receive returning light from the eye to be examined, wherein a longitudinal direction of an image formed by the illumination light on the fundus is approximately parallel to a plane including two or more focal points of the two or more curved mirrors. The method of controlling the fundus imaging apparatus includes: a control step of controlling the illumination optical system so that the image moves in a direction intersecting the longitudinal direction on the fundus; and a capturing step of capturing light receiving result of the returning light in a light-receptive region on a light receiving surface of the image sensor, the light-receptive region corresponding to a position of the image on the fundus.

Still another aspect of the embodiments is a program of causing a computer to execute each step of the method of controlling the fundus imaging apparatus, which is described above.

[EFFECTS OF THE INVENTION]

According to the present invention, a new technique for acquiring higher-quality images of a fundus of an eye to be examined at low cost and with a simple configuration, without requiring advanced optical design can be provided.

[BRIEF EXPLANATION OF THE DRAWINGS]

[FIG. 1] FIG. 1 is a schematic diagram illustrating an example of a configuration of an optical system of a fundus imaging apparatus according to embodiments.

[FIG. 2] FIG. 2 is a schematic diagram illustrating an example of a configuration of the optical system of the fundus imaging apparatus according to the embodiments.

[FIG. 3] FIG. 3 is a schematic diagram illustrating an example of a configuration of an optical system of the fundus imaging apparatus according to the embodiments.

[FIG. 4] FIG. 4 is a schematic diagram for explaining a configuration of the optical system of the fundus imaging apparatus of the embodiments.

[FIG. 5] FIG. 5 is a schematic diagram illustrating an example of a configuration of the optical system of the fundus imaging apparatus according to the embodiments.

[FIG. 6] FIG. 6 is a schematic diagram illustrating an example of a configuration of the optical system of the fundus imaging apparatus according to the embodiments.

[FIG. 7] FIG. 7 is a schematic diagram illustrating an example of a configuration of the optical system of the fundus imaging apparatus according to the embodiments.

[FIG. 8] FIG. 8 is a schematic diagram illustrating an example of a configuration of the optical system of the fundus imaging apparatus according to the embodiments.

[FIG. 9A] FIG. 9A is a schematic diagram for explaining an operation of the fundus imaging apparatus according to the embodiments.

[FIG. 9B] FIG. 9B is a schematic diagram for explaining an operation of the fundus imaging apparatus according to the embodiments.

[FIG. 9C] FIG. 9C is a schematic diagram for explaining an operation of the fundus imaging apparatus according to the embodiments.

[FIG. 10A] FIG. 10A is a schematic diagram for explaining an operation of the fundus imaging apparatus according to a comparative example of the embodiments.

[FIG. 10B] FIG. 10B is a schematic diagram for explaining an operation of the fundus imaging apparatus according to the embodiments.

[FIG. 11] FIG. 11 is a schematic diagram illustrating an example of a configuration of a processing system of the fundus imaging apparatus according to the embodiments.

[FIG. 12] FIG. 12 is a flowchart illustrating an example of the operation of the fundus imaging apparatus according to the embodiments.

[FIG. 13] FIG. 13 is a schematic diagram illustrating an example of a configuration of an optical system of the fundus imaging apparatus according to a first modification example of the embodiments.

[FIG. 14] FIG. 14 is a schematic diagram illustrating an example of a configuration of an optical system of the fundus imaging apparatus according to the first modification example of the embodiments.

[FIG. 15] FIG. 15 is a schematic diagram illustrating an example of a configuration of a processing system of the fundus imaging apparatus according to the first modification example of the embodiments.

[FIG. 16] FIG. 16 is a flowchart illustrating an example of the operation of the fundus imaging apparatus according to the first modification example of the embodiments.

[FIG. 17] FIG. 17 is a schematic diagram illustrating an example of a configuration of an optical system of the fundus imaging apparatus according to a second modification example of the embodiments.

[MODES FOR CARRYING OUT THE INVENTION]

Referring now to the drawings, exemplary embodiments of a fundus imaging apparatus, a method of controlling the fundus imaging apparatus, and a program according to the present invention are described below. Any of the contents of the documents cited in the present specification and arbitrary known techniques may be applied to the embodiments below.

A fundus imaging apparatus according to embodiments includes two or more curved mirrors, and is configured to scan a fundus of an eye to be examined with slit-shaped illumination through the two or more curved mirrors and to receive returning light from the eye to be examined using an image sensor. Each curved mirror has one or more focal points. The two or more curved mirrors are arranged so that two or more focal points of the two or more curved mirrors are arranged on the approximately same plane (common plane of focal points). The fundus imaging apparatus is configured to illuminate the fundus with the illumination light so that a longitudinal direction of a slit image formed by the illumination light projected onto the fundus is approximately parallel to a plane including the two or more focal points, and to scan the fundus with the illumination light in a direction intersecting the longitudinal direction (specifically, orthogonal direction of the longitudinal direction).

Examples of the curved mirror include an ellipsoidal mirror, a paraboloidal mirror, a hyperboloidal mirror, a free-form surface mirror, and a mirror whose reflective surface is represented by higher-order polynomials. The reflective surface of the curved mirror may be a concave-shaped reflective surface or a convex-shaped reflective surface. In other words, examples of the curved mirror include an ellipsoidal concave mirror, an ellipsoidal convex mirror, a paraboloidal concave mirror, a paraboloidal convex mirror, a hyperboloidal concave mirror, a hyperboloidal convex mirror, a free-form surface mirror having a concave-shaped reflective surface, a free-form surface mirror having a convex-shaped reflective surface, a concave mirror whose reflective surface is represented by higher-order polynomials. and a convex mirror whose reflective surface is represented by higher-order polynomials.

In this specification, the focal point may include not only a fixed point uniquely determined by the shape of the curved surface, but also a position where the light beams (light fluxes) reflected by the reflective surface are more focused than at other positions.

The common plane of focal points is preferably a plane on which all focal points of the two or more curved mirrors are arranged. However, the common plane of focal points may be a plane on which two or more focal points, which are obtained by excluding at least one of all the focal points that the two or more curved mirrors have, are arranged.

The slit image projected onto the fundus is, for example, a rectangular-shaped image. However, the shape of the slit image may be a quadrangular shape such as a parallelogram or trapezoid, a polygonal shape, or a shape that extends in a longitudinal direction along one or more predetermined straight lines or curved lines with a predetermined slit width (the slit width need not be constant). In other words, the "slit-shaped" according to the embodiments means that, for example, a light flux cross-sectional shape at the fundus conjugate position is not only rectangular-shaped, but also any other rectangular shape, polygonal shape, or a shape that extends in the longitudinal direction along any straight lines or any curve lines with any width.

The aberration of the slit image on the fundus caused by the curved mirror can be minimized for the direction orthogonal to the arrangement direction of the focal points with reference to the common plane of focal points including the two or more focal points, compared to the arrangement direction of the two or more focal points. This allows to minimize the separation between the slit image projected onto the fundus and the light receivable region (light-receptive region) of the returning light at the fundus conjugate position substantially conjugate optically to the fundus, without being affected by aberrations in the arrangement direction of the two or more focal points of the two or more curved mirrors that relay the slit-shaped illumination light. As a result, there is no longer a need to secure an excessively large region of the light receivable region (light-receptive region) and to include a region for the slit image and its separation. Since the light receivable region (light-receptive region) can be smaller, unnecessary light flux is not captured. Thereby, this allows to improve the contrast of fundus images and to reduce the occurrence of flare and/or ghost, without the need for advanced optical design.

Therefore, higher-quality fundus images of the eye to be examined can be acquired.

A method of controlling the fundus imaging apparatus according to the embodiments includes one or more steps for realizing the processing executed by a processor (computer) in the fundus imaging apparatus according to the embodiments. A program according to the embodiments causes the processor to execute each step of the method of controlling the fundus imaging apparatus according to the embodiments. A recording medium (storage medium) according to the embodiments is a computer readable non-transitory recording medium (storage medium) on which the program according to the embodiments is recorded.

The term "processor" as used herein refers to a circuit such as, for example, a central processing unit (CPU), a graphics processing unit (GPU), an application specific integrated circuit (ASIC), and a programmable logic device (PLD). Examples of PLD include a simple programmable logic device (SPLD), a complex programmable logic device (CPLD), and a field programmable gate array (FPGA). The processor realizes, for example, the function according to the embodiments by reading out a computer program stored in a storage circuit or a storage device and executing the computer program.

Hereinafter, a case where the fundus imaging apparatus according to the embodiments includes two or more ellipsoidal concave mirrors as the two or more curved mirrors and projects rectangular-shaped slit image onto the fundus will mainly described.

Hereinafter, for convenience of explanation, an optical axis direction of the illumination light incident on the eye to be examined is assumed to be a Z direction, a horizontal direction (left-right direction) orthogonal to the Z direction is assumed to be an X direction, and a vertical direction (up-down direction) orthogonal to the Z direction is assumed to be a Y direction.

<Configuration>

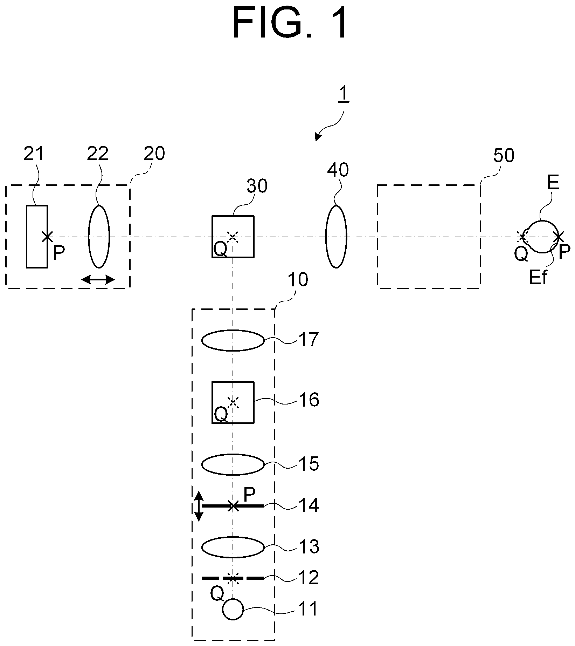

FIG. 1 shows an example of a configuration of an optical system of the fundus imaging apparatus according to the embodiments. It should be noted that, in FIG. 1, a position substantially conjugate optically to the fundus of the eye to be examined is illustrated as a fundus conjugate position P, and a position substantially conjugate optically to an iris (pupil) of the eye to be examined is illustrated as an iris conjugate position (pupil conjugate position) Q.

A fundus imaging apparatus 1 according to the embodiments includes an illumination optical system 10, an imaging optical system 20, a hole mirror 30 as an optical path dividing member, a relay lens 40, and a relay optical system 50. In some embodiments, the relay optical system 50 includes the relay lens 40.

(Illumination optical system 10)

The illumination optical system 10 is configured to generate slit-shaped illumination light (illumination light whose luminous flux cross-sectional shape is slit-shaped), and to project the generated illumination light onto the hole mirror 30, as a slit projection optical system. The illumination optical system 10 includes an illumination light source 11, an iris aperture 12, a relay lens 13, a slit 14, a relay lens 15, an optical scanner 16, and a relay lens 17. In some embodiments, at least one of the relay lenses 13, 15, or 17 includes one or more lenses.

The illumination light source 11 includes a visible light source that generates light in the visible region. For example, the illumination light source 11 generates light having a central wavelength in the wavelength range of 420 nm to 700 nm. This type of illumination light source 11 includes, for example, an LED (Light Emitting Diode), an LD (Laser Diode), a halogen lamp, or a xenon lamp. In some embodiments, the illumination light source 11 includes a white light source or a light source capable of outputting light with each color component of RGB. In some embodiments, the illumination light source 11 includes a light source capable of switching to output the light in infrared region or the light in visible region.

The iris aperture 12 (specifically, its opening(s) (its aperture(s))) is arrangeable at the iris conjugate position Q, which is a position conjugate optically to the iris (pupil) of the eye E to be examined or near the position. In the iris aperture 12, one or more openings are formed at eccentric position(s) from an optical axis of an optical path of light output from the illumination light source 11. The opening(s) formed in the iris aperture 12 defines an incident position (incident shape) of the illumination light on the iris of the eye E to be examined.

In some embodiments, a relative position between the illumination light source 11 and the opening formed in the iris aperture 12 is configured to be changeable. Thereby, the light amount distribution of the light passing through the opening(s) formed in the iris aperture 12 can be changed.

In some embodiments, the illumination light source 11 has a function of the iris aperture 12. In this case, a member that realizes the function of the iris aperture 12 in the illumination light source 11 is placed at the iris conjugate position Q.

The slit 14 (specifically, it opening(s) (its aperture(s))) is arrangeable at the fundus conjugate position P, which is a position substantially conjugate optically to the fundus Ef of the eye E to be examined or near the position. The opening(s) formed in the slit 14 defines a shape of an illumination region (irradiated pattern shape) on the fundus Ef of the eye E to be examined. The illumination light emitted from the illumination light source 11 passes through the slit 14 and is projected onto the fundus Ef as slit-shaped illumination light.

The slit 14 is movable in the optical axis direction of the illumination optical system using a movement mechanism (not show) (specifically, movement mechanism 14D described below). This allows to move the position of the slit 14 in accordance with the state of the eye E to be examined (specifically, the dioptric power (refractive power) or the shape of the fundus Ef).

In some embodiments, the slit 14 is configured so that at least one of the position(s) of the opening(s) or the shape of the opening(s) can be changed in accordance with the state of the eye E to be examined without being moved in the optical axis direction. The function of the slit 14 with these functions is, for example, realized by a liquid crystal shutter.

The optical scanner 16 (specifically, its deflection surfaces) is arrangeable at the iris conjugate position Q, which is a position conjugate optically to the iris (pupil) of the eye E to be examined or near the position. The optical scanner 16 is a uniaxial optical scanner that deflects an orientation of the deflection surface in a predetermined deflection direction. The optical scanner 16 one-dimensionally deflects the slit-shaped illumination light formed by passing through the slit 14. The optical scanner 16 deflects the illumination light in a direction intersecting (specifically, orthogonal to) the longitudinal direction of the slit image formed by the slit-shaped illumination light projected onto the fundus Ef. Thereby, the slit image moves in the direction intersecting the longitudinal direction of the slit image (scan direction).

The optical scanner 16 includes, for example, a galvanometer scanner, a MEMS (Micro Electro Mechanical System) scanner, a polygon mirror, or a resonant scanner. For example, the optical scanner 16 includes the galvanometer scanner that deflects the illumination light within a predetermined deflection angular range with reference to a predetermined deflection direction.

In some embodiments, the optical scanner 16 is a biaxial optical scanner that two-dimensionally deflects the slit-shaped illumination light. For example, the optical scanner 16 includes a first scanner and a second scanner. The first scanner deflects the illumination light so as to move the illumination region on the fundus Ef in a horizontal direction orthogonal to the optical axis of the illumination optical system 10. The second scanner deflects the illumination light deflected by the first scanner so as to move the illumination region on the fundus Ef in a vertical direction orthogonal to the optical axis of the illumination optical system 10.

The light from the illumination light source 11 that has passed through the opening(s) formed in the iris aperture 12 is transmitted through the relay lens 13, passes through the opening(s) formed in the slit 14, and is output as the slit-shaped illumination light. The slit-shaped illumination light emitted from the slit 14 is transmitted through the relay lens 15, is deflected one-dimensionally by the optical scanner 16, is transmitted through the relay lens 17, and is guided to the hole mirror 30.

In some embodiments, the illumination optical system 10 includes a projector with a light source, and the projector emits the slit-shaped illumination light. In this case, the projector is provided instead of the illumination light source 11, the iris aperture 12, the relay lens 13, and the slit 14 in FIG. 1. Examples of the projector include an LCD (Liquid Crystal Display) type projector using a transmissive LCD panel, an LCOS (Liquid Crystal On Silicon) type projector using a reflective LCD panel, a DLP (Digital Light Processing) (registered trademark) type projector using a DMD (Digital Mirror Device).

(Hole mirror 30)

The hole mirror 30 (specifically, its hole part described below) is arrangeable at the iris conjugate position Q. The hole mirror 30 is an optical path dividing member that spatially divides the optical path of the illumination light and the optical path of the returning light from the eye E to be examined, onto which the illumination light has been irradiated.

In the hole mirror 30, the hole part, through which the optical axis passes, is formed in a central part of a base substance, and a reflective surface, which reflects the light, is provided around a peripheral part of the central part. In FIG. 1, the hole mirror 30, which is positioned so that the optical axis of the imaging optical system 20 passes through the hole part, is configured to reflect the illumination light from the illumination optical system 10 on the reflective surface provided in the peripheral part to guide the reflected illumination light to the eye E to be examined. In some embodiments, the hole mirror 30, which is positioned so that the optical axis of the illumination optical system 10 passes through the hole part, is configured to reflect the returning light from the eye E to be examined on the reflective surface provided in the peripheral part to guide the reflected returning light to the imaging optical system 20. In some embodiments, in the hole mirror 30, the hole part, through which the optical axis passes, is formed in one side of the base substance, and the reflective surface, which reflects the light, is provided in the other side of the base substance. In this case, the light passing through the hole part is guided to the imaging optical system 20, and the light reflected by the reflective surface is guided to the eye E to be examined. Alternatively, conversely, the light passing through the hole part is guided to the eye E to be examined, and the light reflected by the reflective surface is guided to the imaging optical system 20.

The illumination light from the illumination optical system 10 is reflected on the reflective surface on the peripheral part of the hole part formed in the hole mirror 30, and is guided to the relay lens 40. The returning light from the eye E to be examined is transmitted through the relay lens 40, passes through the hole part formed in the hole mirror 30, and is guided to the imaging optical system 20.

In some embodiments, a beam splitter, a half mirror, a multifaceted mirror, or a dichroic mirror is placed instead of the hole mirror 30.

(Imaging optical system 20)

The imaging optical system 20 is configured to receive the returning light from the eye E to be examined, which has passed through the hole part formed in the hole mirror 30, as a slit light receiving optical system. Here, the returning light from the eye E to be examined is scattered light (reflected light) of the illumination light incident on the eye E to be examined. In some embodiments, the returning light from the eye E to be examined includes scattered light (reflected light) of the illumination light incident on the eye E to be examined and fluorescence and its scattered light using the illumination light incident on the eye E to be examined as excitation light.

The imaging optical system 20 includes an image sensor 21 and a focusing lens 22.

The focusing lens 22 is movable in the optical axis direction of the imaging optical system 20 using a movement mechanism (not show) (specifically, movement mechanism 22D described below). This allows to adapt the state of the eye E to be examined and to image the returning light from the eye E to be examined on the light receiving surface of the image sensor 21.

The image sensor 21 realizes the function of the two-dimensional image sensor as a pixelated photodetector. The light receiving surface (detecting surface, imaging surface) of the image sensor 21 is arrangeable at the fundus conjugate position P. The image sensor 21 is configured to be capable of setting a light receivable region (light-receptive region) that can be virtually moved at the fundus conjugate position P.

For example, the light receiving result(s) acquired by the image sensor 21 is/are captured and read out using a rolling shutter system (method). In some embodiments, the light receiving result(s) acquired by the image sensor 21 is/are captured and read out using a global shutter system (method) in which the light receivable region can be changed or be moved. In some embodiments, the controller described below performs readout control of the light receiving result(s) by controlling the image sensor 21. In some embodiments, the image sensor 21 can automatically output the light receiving result(s) for a predetermined number of lines, along with information indicating the light receiving position(s).

The image sensor 21 with such a configuration includes a CMOS (Complementary metal oxide semiconductor) image sensor. In this case, the image sensor 21 includes a plurality of pixels (light receiving elements). The plurality of pixels includes a plurality of pixel groups arranged in a column direction. Each of the plurality of pixel groups includes pixels arranged in a row direction. Specifically, the image sensor 21 includes a plurality of pixels arranged two-dimensionally, a plurality of vertical signal lines, and a horizontal signal line.

In some embodiments, the image sensor 21 is a CCD (Charge Coupled Device) image sensor, for example.

By capturing (reading out) the light receiving result(s) of the returning light using the rolling shutter system for such an image sensor 21, the image in the light receivable region corresponding to a desired virtual opening shape extending in the row direction is acquired. Such control is disclosed in, for example, Patent Document 2 or U.S. Patent No. 8237835.

The returning light from the eye E to be examined, onto which the illumination light from the illumination optical system 10 has irradiated, passes through the hole part formed in the hole mirror 30, is transmitted through the focusing lens 22, and forms an image on the light receiving surface of the image sensor 21.

In some embodiments, one or more relay lenses are disposed between the focusing lens 22 and the image sensor 21.

(Relay optical system 50)

The relay optical system 50 is disposed between the relay lens 40 and the eye E to be examined. The relay optical system 50 is configured to relay the slit image, which is formed by the slit-shaped illumination light generated by the illumination optical system 10, to the fundus Ef of the eye E to be examined. The relay optical system 50 includes a reflective optical system with an angle-of-view extension function. In this case, the relay optical system 50 may further include a refractive optical system such as an aberration correcting lens. In some embodiments, the relay optical system 50 does not have the angle-of-view extension function.

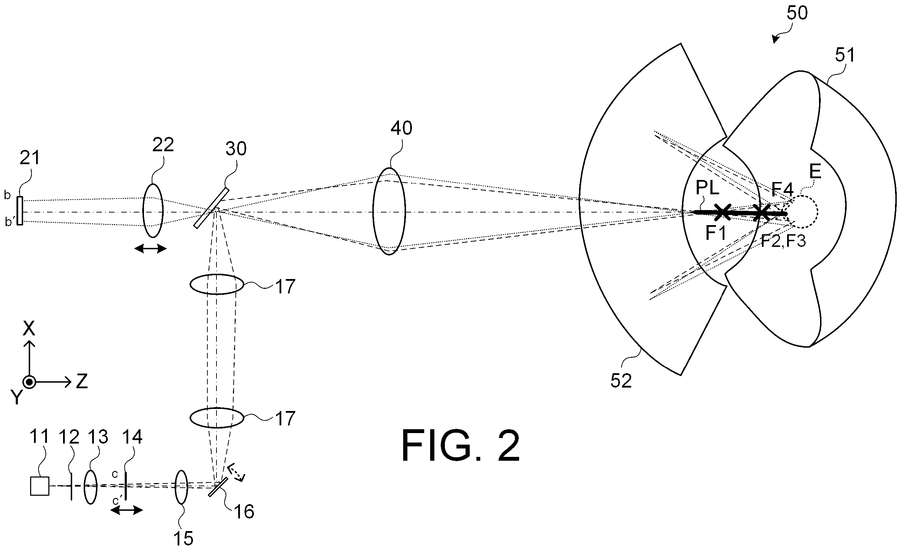

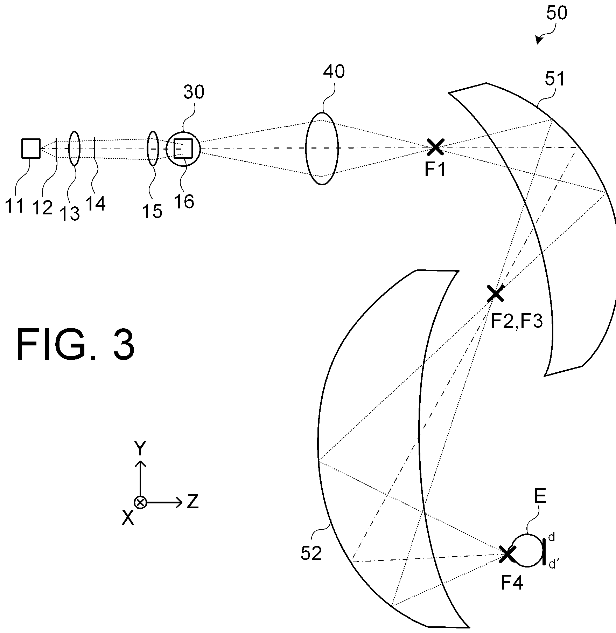

FIG. 2 and FIG. 3 show examples of a configuration of the relay optical system 50 in FIG. 1. FIG. 2 schematically represents an example of the configuration of the relay optical system 50 when viewed from the top (X-Z plane). FIG. 3 schematically represents an example of the configuration of the relay optical system 50 when viewed from the side (Y-Z plane). In FIG. 2 and FIG. 3, like parts are designated by like reference numerals as in FIG. 1 and repetitious description of such parts may not be provided.

As shown in FIG. 2 and FIG. 3, the relay optical system 50 includes a first ellipsoidal concave mirror 51 and a second ellipsoidal concave mirror 52.

(First ellipsoidal concave mirror 51)

A reflective surface of the first ellipsoidal concave mirror 51 is a concave-shaped elliptical surface. The first ellipsoidal concave mirror 51 is an example of the curved mirror or the concave mirror.

The first ellipsoidal concave mirror 51 has two optically conjugate focal points (first focal point F1, second focal point F2). The relay lens 40 is positioned so that the iris conjugate position (pupil conjugate position) Q substantially coincides with the first focal point F1.

(Second ellipsoidal concave mirror 52)

A reflective surface of the second ellipsoidal concave mirror 52 is a concave-shaped elliptical surface. The second ellipsoidal concave mirror 52 is an example of the curved mirror or the concave mirror.

The second ellipsoidal concave mirror 52 has two optically conjugate focal points (first focal point F3, second focal point F4). The second ellipsoidal concave mirror 52 is positioned so that the first focal point F3 substantially coincides with the second focal point F2 of the first ellipsoidal concave mirror 51. In some embodiments, the second ellipsoidal concave mirror 52 is positioned so that the first focal point F3 substantially coincides with a position conjugate optically to the second focal point F2 of the first ellipsoidal concave mirror 51 (conjugate position of the second focal point F2) or near the position conjugate optically to the second focal point F2. The eye E to be examined (iris) is positioned at the second focal point F4 of the second ellipsoidal concave mirror 52. In other words, the second ellipsoidal concave mirror 52 is positioned so that the second focal point F4 substantially coincides with eye to be examined position where the eye E to be examined is positioned.

Furthermore, as shown in FIG. 2, the first ellipsoidal concave mirror 51 and the second ellipsoidal concave mirror 52 are positioned so that the two focal points (first focal point F1, second focal point F2) of the first ellipsoidal concave mirror 51 and the two focal points (first focal point F3, second focal point F4) of the second ellipsoidal concave mirror 52 are positioned on substantially the same plane (common plane PL of focal points). In addition, the slit 14 is disposed so that the longitudinal direction of the slit image formed by the slit-shaped illumination light projected onto the fundus Ef is approximately parallel to the common plane PL of focal points. In some embodiments, the slit 14 is disposed so that the longitudinal direction of the slit image formed by the slit-shaped illumination light projected onto the fundus Ef is positioned on the common plane PL of focal points, in a state where the position matching has been performed.

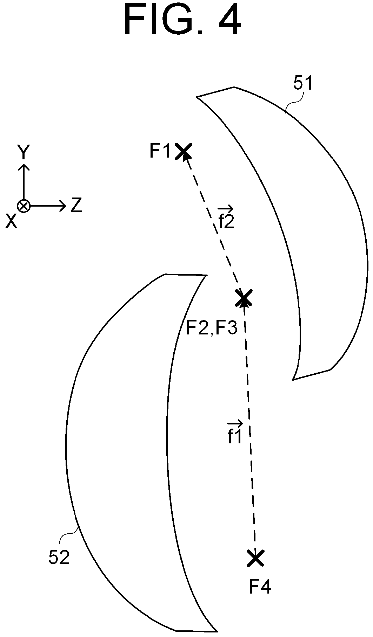

FIG. 4 schematically shows a relationship between the longitudinal direction of the slit image and the focal points of the first ellipsoidal concave mirror 51 and the second ellipsoidal concave mirror 52. In FIG. 4, like reference numerals designate like parts as in FIG. 3, and the redundant explanation may be omitted as appropriate.

Here, the longitudinal direction of the slit image on the fundus Ef is denoted as a vector "L", and the direction orthogonal (perpendicular) to the longitudinal direction of the slit image is denoted as a vector "S". Further, a vector from the second focal point F4 to the first focal point F3 of the second ellipsoidal concave mirror 52 is denoted as a vector "f1", and a vector from the second focal point F2 to the first focal point F1 of the first ellipsoidal concave mirror 51 is denoted as a vector "f2". When denoting the normal vector of the common plane PL of focal points as a vector "n", the vector "n" is expressed as in Equation (1). In this case, the longitudinal direction of the slit image and the focal points of the first ellipsoidal concave mirror 51 and the second ellipsoidal concave mirror 52 are arranged so that the vectors "S" described above and the vector "n" are parallel to each other, as shown in Equation (2).

[Equation 1]

$$\vec{n} = \overrightarrow{f1} \times \overrightarrow{f2} \qquad\qquad (1)$$

$$\vec{S} \parallel \vec{n} \qquad\qquad (2)$$

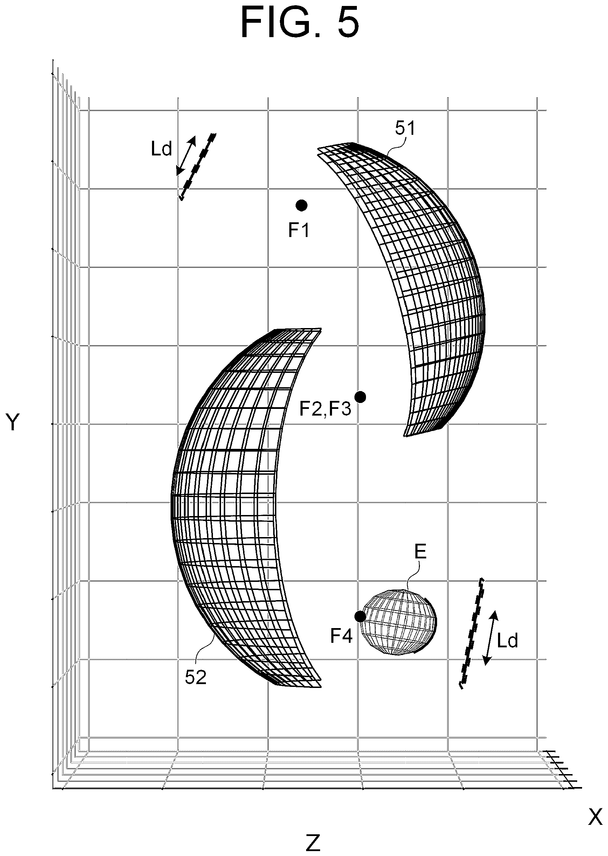

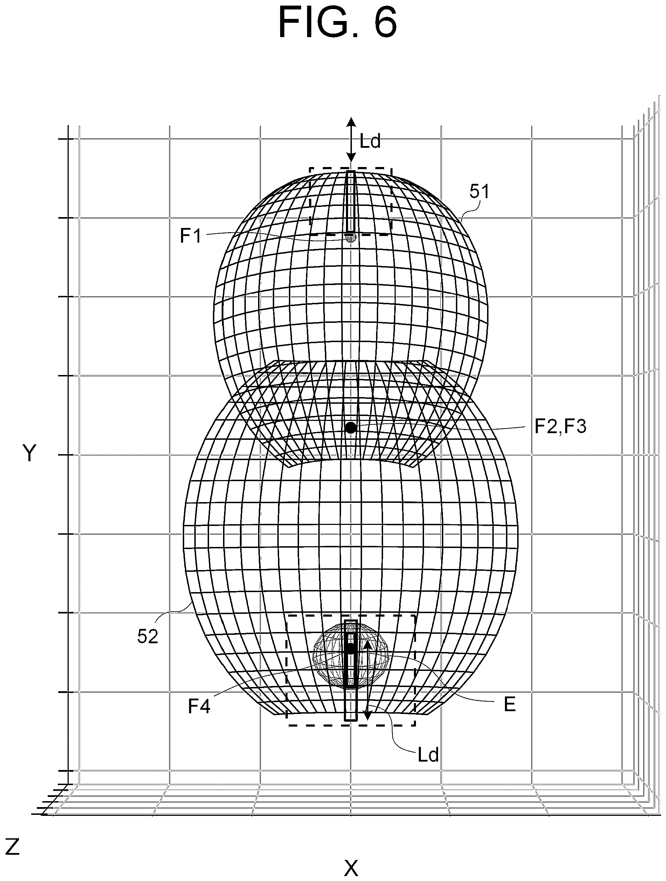

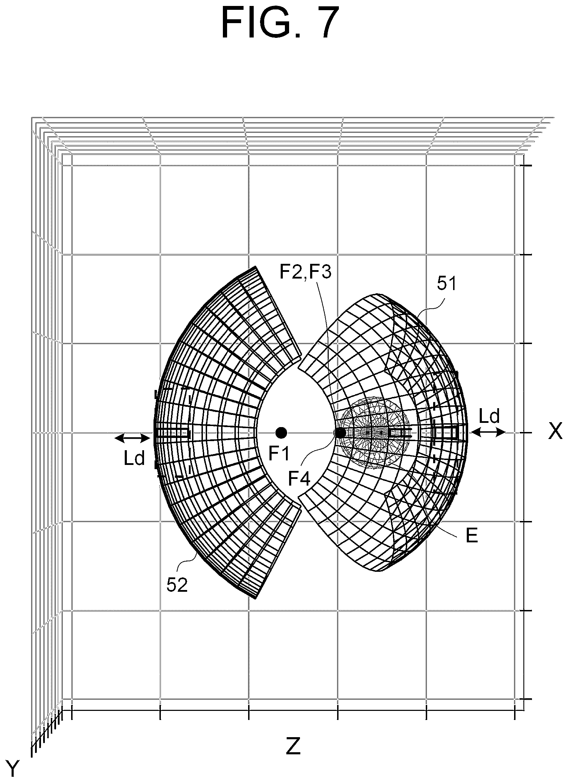

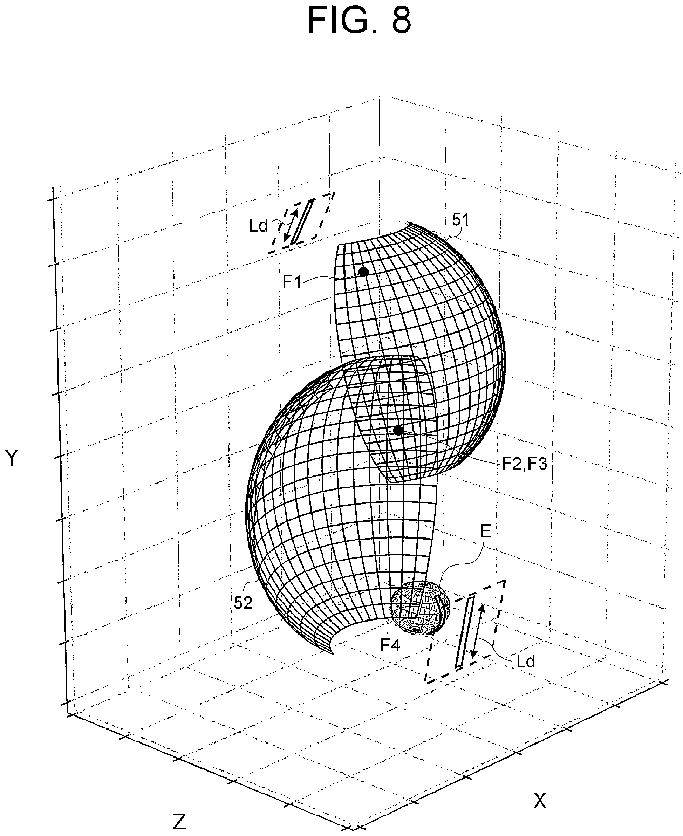

FIG. 5, FIG. 6 FIG. 7, and FIG. 8 show a relationship among the focal points of the first ellipsoidal concave mirror 51 according to the embodiments, the focal points of the second ellipsoidal concave mirror 52 according to the embodiments, and the slit image projected onto the fundus Ef. FIG. 5, FIG. 6, FIG. 7, and FIG. 8 represent wireframes indicating an example of a three-dimensional positional relationship among the first ellipsoidal concave mirror 51, the second ellipsoidal concave mirror 52, and the eye E to be examined. In FIG. 5, FIG. 6, FIG. 7, and FIG. 8, the longitudinal direction (Ld) of the slit image incident on the first ellipsoidal concave mirror 51, and the longitudinal direction (Ld) of the slit image projected onto the fundus Ef of the eye E to be examined are illustrated.

FIG. 5 represents a wireframe indicating a relationship among the first ellipsoidal concave mirror 51 and the second ellipsoidal concave mirror 52 and the longitudinal direction Ld of the slit image, when viewed from the lateral (X) direction. FIG. 6 represents a wireframe indicating a relationship among the first ellipsoidal concave mirror 51 and the second ellipsoidal concave mirror 52 and the longitudinal direction Ld of the slit image, when viewed from a side facing the reflective surface of the second ellipsoidal concave mirror 52. FIG. 7 represents a wireframe indicating a relationship among the first ellipsoidal concave mirror 51 and the second ellipsoidal concave mirror 52 and the longitudinal direction Ld of the slit image, when viewed from the upper (top) direction (Y direction). FIG. 8 represents a wireframe indicating a relationship among the first ellipsoidal concave mirror 51 and the second ellipsoidal concave mirror 52 and the longitudinal direction Ld of the slit image, when viewed from an oblique direction. In FIG. 5, FIG. 6, FIG. 7, and FIG. 8, like reference numerals designate like parts as in FIG. 2 and FIG. 3. The same description may not be repeated.

For example, as shown in FIG. 2, the first focal point F1 of the first ellipsoidal concave mirror 51, the second focal point F2 of the first ellipsoidal concave mirror 51, the first focal point F3 of the second ellipsoidal concave mirror 52, and the second focal point F4 of the second ellipsoidal concave mirror 52 are positioned on the same plane (common plane PL of focal points). As described above, the illumination optical system 10 projects the slit image, whose longitudinal direction is parallel to the common plane of focal points described above, onto the fundus Ef of the eye E to be examined , by entering the slit-shaped illumination light into the first ellipsoidal concave mirror 51 so that the longitudinal direction Ld of the slit image is approximately parallel to the common plane of focal points described above. More specifically, the illumination optical system 10 projects the slit image, whose longitudinal direction Ld is parallel to the common plane of focal points described above, onto the fundus Ef of the eye E to be examined, by entering the slit-shaped illumination light, in which the longitudinal direction Ld of the slit image is arranged on the common plane of focal points described above. The illumination optical system 10 scan the fundus Ef with the illumination light by scanning this slit image in the direction orthogonal to the longitudinal direction Ld using the optical scanner 16.

In the configuration as described above, the slit-shaped illumination light from the illumination optical system 10 is reflected on the reflective surface of the first ellipsoidal concave mirror 51, and is guided to the second focal point F2 of the first ellipsoidal concave mirror 51. The illumination light that has been guided to the second focal point F2 (first focal point F3) is guided to the reflective surface of the second ellipsoidal concave mirror 52, is reflected on this reflective surface, and is guided to the eye E to be examined arranged at the second focal point F4 of the second ellipsoidal concave mirror 52.

The illumination light that has been guided to the eye E to be examined enters the eye through the pupil and is irradiated onto the fundus Ef. The returning light from the eye E to be examined is emitted outside of the eye E to be examined through the pupil, travels in the same path as the outward path in the opposite direction, and is guided to the first focal point F1 of the first ellipsoidal concave mirror 51. The returning light that has been guided to the first focal point F1 passes through the hole part formed in the hole mirror 30, and is guided to the imaging optical system 20, as described above. In the imaging optical system 20, the returning light is received on the light receiving surface of the image sensor 21, and the light receiving result of the returning light in the light receivable region set on the light receiving surface is captured.

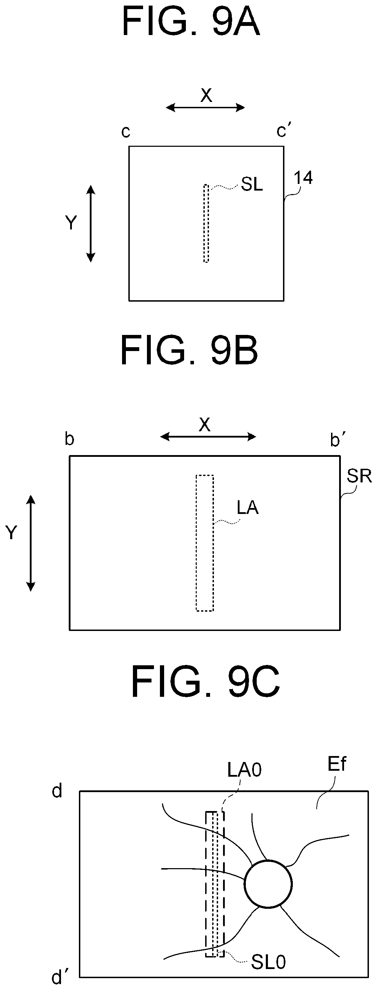

FIG. 9A, FIG. 9B, and FIG. 9C schematically show the slit-shaped illumination light and the light receivable region on the image sensor 21. FIG. 9A schematically represents the opening SL formed in the slit 14. FIG. 9B schematically represents a light receivable region LA on the light receiving surface SR of the image sensor 21. FIG. 9C schematically represents a slit image SL0 projected onto the fundus Ef and a light receivable region LA0 on the light receiving surface of the image sensor 21.

Light from the illumination light source 11 that has passed through the opening SL shown in FIG. 9A is emitted as the slit-shaped illumination light whose longitudinal direction follows the direction orthogonal to the c-c' direction in FIG. 2. The slit-shaped illumination light is deflected by the optical scanner 16, as described above, and enters the eye through the pupil of the eye E to be examined via the first ellipsoidal concave mirror 51 and the second ellipsoidal concave mirror 52, and is projected onto the fundus Ef. The optical scanner 16 scans so that the illumination region defined by the slit image SL0 moves in a direction orthogonal to the longitudinal direction (d-d' direction in FIG. 3) of the slit image SL0 on the fundus Ef , as shown in FIG. 9C.

As shown in FIG. 9B, the image sensor 21 is configured to be capable of virtually moving the light receivable region LA on the light receiving surface SR. Thereby, the reduction of contrast, the occurrence of flare and/or ghost, which are caused by the reception of unwanted light from the eye E to be examined, can be prevented. The image sensor 21 changes the position of the light receivable region LA on the light receiving surface SR in synchronization with the movement of the illumination region of the illumination light on the fundus Ef. Specifically, as shown in FIG. 9C, the light receivable region LA of the image sensor 21 is set in accordance with the light receivable region LA0 on the fundus Ef so as to include a range of the slit image SL0 on the fundus Ef. In other words, the light receivable region LA is set in a strip shape whose longitudinal direction follows a direction orthogonal to the b-b' direction in FIG. 2. And, the image sensor 21 sequentially moves the light receivable region LA on the light receiving surface SR in accordance with the light receivable region LA0, in synchronization with the movement of the slit image SL0 on the fundus Ef so as to include the range of the slit image SL0.

Idealistically, it is desirable that the slit image SL0 and the light receivable region LA0 coincide with each other on the fundus Ef. However, due to aberrations in the optical system, the light receivable region LA0 must be wider than the slit image SL0.

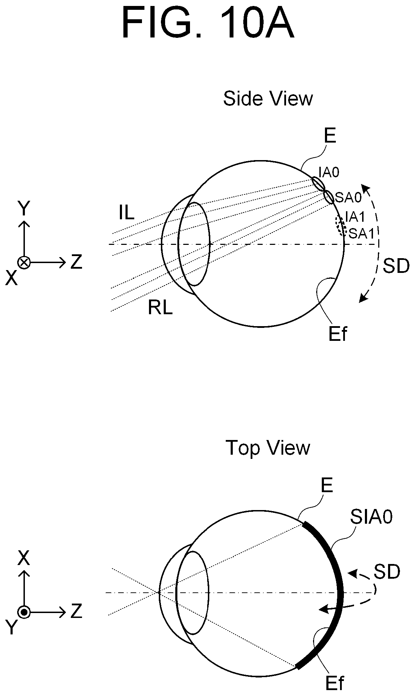

FIG. 10A shows a diagram for explaining an operation of a fundus imaging apparatus according to a comparative example of the embodiments. FIG. 10A schematically represents a side view (Side View) of the eye E to be examined scanned using the slit-shaped illumination light when viewed from the X direction, and a top view (Top View) of the eye E to be examined scanned using the slit-shaped illumination light when viewed from the Y direction.

In the comparative example of the embodiments, the longitudinal direction of the slit image is assumed to be orthogonal to the common plane of focal points described above, and the fundus Ef is assumed to be scanned in the direction perpendicular to the longitudinal direction of the slit image (in other words, the common plane of focal points described above is assumed to be parallel to or coincide with the scan direction SD).

In the present comparative example, the slit image SIA0 projected onto the fundus Ef is moved along the scan direction SD. In this case, the illumination region IA0 (IA1) illuminated by the illumination light flux IL and the light-receptive region SA0 (SA1) on the light receiving surface of image sensor 21 are separated as they move away from the optical axis, due to the aberration of the optical system. Therefore, in order to suppress the decrease in the light receiving amount of the light receiving light flux RL from the light-receptive region SA0, the light receivable region LA0 in FIG. 9C must be wider than the slit image SL0.

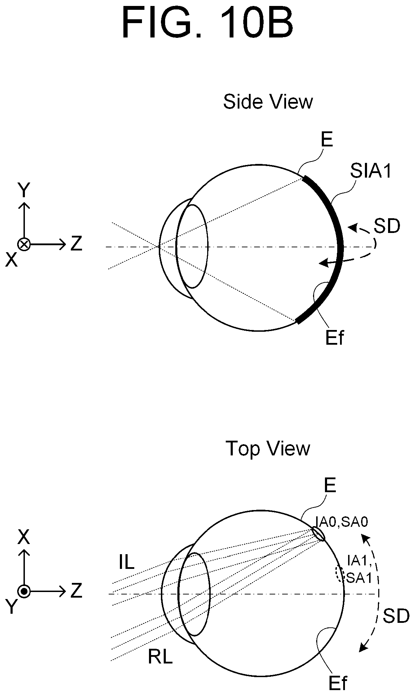

FIG. 10B shows a diagram for explaining an operation of the fundus imaging apparatus 1 according to the embodiments. FIG. 10B schematically represents a side view (Side View) of the eye E to be examined scanned using the slit-shaped illumination light when viewed from the X direction, and a top view (Top View) of the eye E to be examined scanned using the slit-shaped illumination light when viewed from the Y direction, in the same way as in FIG. 10A.

In the embodiments, as described above, the longitudinal direction of the slit image coincides with or is approximately parallel to a direction the common plane of focal points described above, and the fundus Ef is scanned in the direction orthogonal to the longitudinal direction of the slit image.

In other words, in the embodiments, the slit image SIA1 projected onto the fundus Ef is moved along the scan direction SD. The aberration of the slit image on the fundus caused by the curved mirror such as an ellipsoidal concave mirror can be minimized for the direction orthogonal to the arrangement direction of the focal points with reference to the common plane of focal points including the two or more focal points, compared to the arrangement direction of the two or more focal points. Therefore, the separation between the illumination region IA0 (IA1) illuminated by the illumination light flux IL and the light-receptive region SA0 (SA1) can be minimized, even when they are far from the optical axis. Therefore, there is no longer a need to suppress the decrease in the light receiving amount of the light receiving light flux RL from the light-receptive region SA0. As a result, the separation between the light receivable region LA0 and the slit image SL0 in FIG. 9C can be minimized. Thereby, contrast can be improved, and the occurrence of flare and/or ghost can be significantly reduced.

It should be noted that, in FIG. 10A and FIG. 10B, each of the illumination light flux IL and the imaging light flux RL is illustrated as passing through the upper and lower iris. However, the configuration are not limited to this. For example, it is sufficient that the illumination light flux IL and the imaging light flux RL are spatially separated in the iris. The illumination light flux IL may pass through the peripheral part, and the imaging light flux RL may pass through the central part.

In some embodiments, at least one of the first ellipsoidal concave mirror 51 or the second ellipsoidal concave mirror 52 is a convex mirror whose reflective surface is formed in a convex shape (for example, ellipsoidal convex mirror). In some embodiments, at least one of the first ellipsoidal concave mirror 51 or the second ellipsoidal concave mirror 52 is a curved mirror whose reflective surface is a free-form surface.

In addition to the configuration shown in FIG. 1, the fundus imaging apparatus 1 may be provided with an alignment optical system for performing position matching between the eye E to be examined and the optical system.

Further, the fundus imaging apparatus 1 may have a configuration for providing a function associated with the inspection. For example, the fundus imaging apparatus 1 may be provided with a fixation optical system for projecting a visual target (fixation target) for fixating the eye E to be examined onto the fundus Ef of the eye E to be examined. Further, the fundus imaging apparatus 1 may be provided with any element or unit, such as a member for supporting the face of the examinee (chin rest, forehead pad, etc.).

In the present embodiment, the configuration according to the embodiments is configured to be capable of focusing on the fundus Ef of the eye E to be examined, using the movement of the slit 14 and/or the movement of the focusing lens 22. However, the configuration according to the embodiments is not limited to this. In some embodiments, the fundus imaging apparatus is configured to be capable of moving the entire optical system (illumination optical system 10, imaging optical system 20, hole mirror 30, and relay lens 40, or illumination optical system 10, imaging optical system 20, hole mirror 30, relay lens 40, first ellipsoidal concave mirror 51, and second ellipsoidal concave mirror 52) using a movement mechanism not shown.

The hole mirror 30 is an example of the "optical path dividing member" according to the embodiments. The first focal point F3 of the second ellipsoidal concave mirror 52 is an example of the "third focal point of the second ellipsoidal concave mirror" according to the embodiments. The second focal point F4 of the second ellipsoidal concave mirror 52 is an example of the "fourth focal point of the second ellipsoidal concave mirror" according to the embodiments.

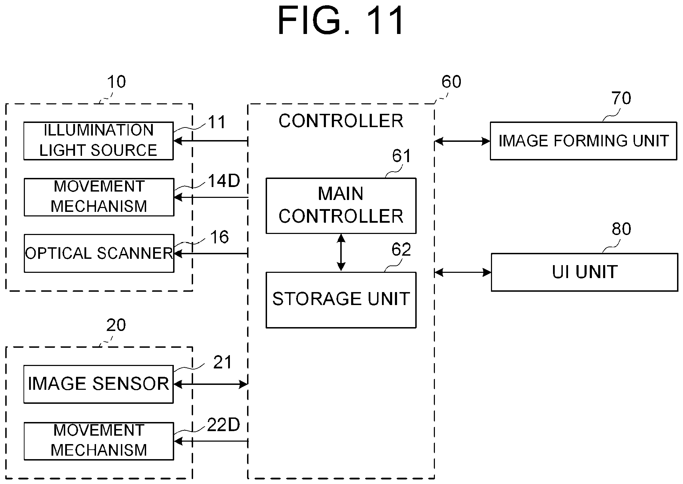

FIG. 11 shows an example of a configuration of a processing system of the fundus imaging apparatus 1 according to the embodiments. In FIG. 11, parts similar to those in FIG. 1, FIG. 2, and FIG. 3 are denoted by the same reference symbols, and description thereof is omitted as appropriate.

The processing system of the fundus imaging apparatus 1 is configured with a controller 60 as a center. The controller 60 controls each part of the fundus imaging apparatus 1.

The controller 60 includes a main controller 61 and a storage unit 62. The functions of the main controller 61 are realized by a processor, for example. The storage unit 62 stores, in advance, computer programs for controlling the fundus imaging apparatus 1. Examples of the computer programs include an illumination light source control program, an image sensor control program, an optical scanner control program, an image forming program, and a program for user interface. The main controller 61 operates according to such the computer program(s), and thereby the controller 60 performs the control processing.

(Main controller 61)

The main controller 61 controls each of the illumination optical system 10, the imaging optical system 20, the image forming unit 70, and the user interface (UI) unit 80.

Examples of the control for the illumination optical system 10 include control for the illumination light source 11, control for the movement mechanism 14D, and control for the optical scanner 16.

Examples of the control for the illumination light source 11 include turning the light source on and off, adjustment of the light amount, and adjustment of an aperture.

The movement mechanism 14D moves the slit 14 in the optical axis direction of the illumination optical system 10. The main controller 61 outputs control signal(s) to the movement mechanism 14D to move the slit 14 in a direction corresponding to the control signal(s) by the amount of movement corresponding to the control signal(s). For example, the fundus imaging apparatus 1 is provided with an actuator that generates a driving force for moving the movement mechanism 14D and a transmission mechanism that transmits the driving force from the actuator to the movement mechanism 14D. The actuator includes a pulse motor, for example. The transmission mechanism includes a combination of gears, a rack and pinion, and the like, for example. The movement mechanism 14D receives the driving force generated by the actuator controlled by the main controller 61 from the transmission mechanism, and moves the slit 14 in the optical axis direction.

The control for the optical scanner 16 includes control of the angle of the deflection surface deflecting the illumination light. By controlling the angle of the deflection surface, the deflection direction (scan direction) of illumination light can be controlled. By controlling an angular range of the deflection surface, the scan range (scan start position and scan end position) can be controlled. By controlling the speed of changing the angle of deflection surface, the scan speed can be controlled.

In some embodiments, in case that the orientation of the common plane of focal points is changeable, the optical scanner 16 is configured to change the deflection direction of the illumination light in conjunction with the change in the deflected orientation of the common plane of focal points.

Examples of the control for the imaging optical system 20 include control for the image sensor 21, and control for the movement mechanism 22D.

The control for the image sensor 21 includes control of setting the light receivable region on the light receiving surface, and control for reading out the light receiving result(s) using the rolling shutter system (for example, setting of light receiving size corresponding to the size of the illumination pattern, or the like). Further, the control for the image sensor 21 includes the reset control, the exposure control, the charge transfer control, and the output control.

The movement mechanism 22D moves the focusing lens 22 in the optical axis direction of the imaging optical system 20. The main controller 61 outputs control signal(s) to the movement mechanism 22D to move the focusing lens 22 in a direction corresponding to the control signal(s) by the amount of movement corresponding to the control signal(s). For example, the fundus imaging apparatus 1 is provided with an actuator that generates a driving force for moving the movement mechanism 22D and a transmission mechanism that transmits the driving force from the actuator to the movement mechanism 22D. The actuator includes a pulse motor, for example. The transmission mechanism includes a combination of gears, a rack and pinion, and the like, for example. The movement mechanism 22D receives the driving force generated by the actuator controlled by the main controller 61 from the transmission mechanism, and moves the focusing lens 22 in the optical axis direction.

Examples of the control for the image forming unit 70 include image forming control for forming images of the eye E to be examined from the light receiving result(s) obtained using the image sensor 21.

Examples of the control for the UI unit 80 include control for a display device and control for an operation device (input device).

(Storage unit 62)

The storage unit 62 stores various types of data. Examples of the data stored in the storage unit 62 include light receiving result(s) obtained by the image sensor 21, image data of an image formed by the image forming unit 70, and information on eye to be examined. The information on the eye to be examined includes information on the examinee such as patient ID and name, and information on the eye to be examined such as identification information of the left/right eye.

The storage unit 62 further stores various types of programs and data to run the fundus imaging apparatus 1.

(Image forming unit 70)

The image forming unit 70 can form the light receiving image (fundus image) corresponding to the arbitrary light receivable region, based on the light receiving result(s) read out from the image sensor 21 using the rolling shutter system. The image forming unit 70 can sequentially form light receiving light images corresponding to the light receivable regions and form an image of the eye E to be examined from a plurality of the formed light receiving images. The various images (the various image data) formed by the image forming unit 70 are stored in the storage unit 62, for example.

For example, the image forming unit 70 includes a processor, and executes the function described above by performing processing corresponding to the program(s) stored in the storage unit or the like.

(UI unit 80)

The UI unit 80 has a function for exchanging information between a user and the fundus imaging apparatus 1. The UI unit 80 includes the display device and the operation device. The display device may include a display unit, and it may include another display device. The display device displays various information. The display device includes a liquid crystal display, for example. The display device displays the above information under the control of the main controller 61. Examples of the information displayed on the display device include information corresponding to the control result by the controller 60, information (image) corresponding to the calculation result by the image forming unit 70. The operation device includes various hardware keys and/or various software keys. Upon receiving the operation content for the operation device, the main controller 61 can output a control signal corresponding to the operation content to each part of the fundus imaging apparatus. At least a part of the display device and at least a part of the operation device may be configured integrally. One example of this is the touch panel display.

<Operation>

Next, the operation of the fundus imaging apparatus 1 according to the embodiments will be described.

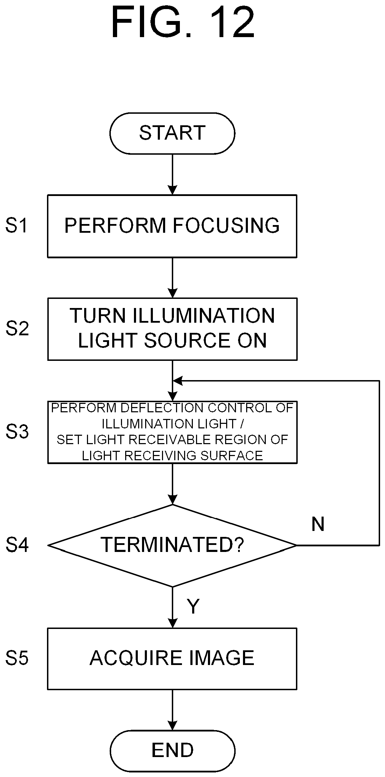

FIG. 12 shows an example of the operation of the fundus imaging apparatus 1 according to the embodiments. FIG. 12 represents a flowchart of the example of the operation of the fundus imaging apparatus 1 according to the embodiments. The storage unit 62 stores computer program(s) for realizing the processing shown in FIG. 12. The main controller 61 operates according to the computer program, and thereby the main controller 61 performs the processing shown in FIG. 12.

In FIG. 12, it is assumed that the eye E to be examined has been arranged at a predetermined eye to be examined position (the second focal point F4 of the second ellipsoidal concave mirror 52), and that the illumination range (scan range) of the illumination light on the fundus Ef has been determined in advance.

(S1: Perform focusing)

First, the main controller 61 controls at least one of the movement mechanism 14D or the movement mechanism 22D to adjust the focal position of the illumination optical system 10 and the focal position of the imaging optical system 20 with respect to the fundus Ef of the eye E to be examined.

(S2: Turn illumination light source on)

Next, the main controller 61 controls the illumination light source 11 to turn the illumination light source 11 on.

The light output from the illumination light source 11 passes through the opening(s) formed in the iris aperture 12, is transmitted through the relay lens 13, passes through the opening(s) formed in the slit 14, is transmitted through the relay lens 15, and is guided to the hole mirror 30 as the slit-shaped illumination light via the optical scanner 16 and the relay lens 17.

(S3: Perform deflection control of illumination light / Set light receivable region of light receiving surface)

Subsequently, the main controller 61 controls the optical scanner 16 to set the orientation of the deflection surface in a predetermined deflection direction in order to illuminate a predetermined illumination range (scan range), and starts deflection control of the illumination light that sequentially changes the orientation of the deflection surface within a predetermined deflection angular range. In other words, the main controller 61 starts scanning of the illumination light onto the fundus Ef.

In some embodiments, the main controller 61 controls the optical scanner 16 to perform deflection control of the illumination light in synchronization with the movement of the light receivable region that can be arbitrarily set on the image sensor 21.

In some embodiments, the main controller 61 controls the image sensor 21 to set the light receivable region of the returning light on the light receiving surface. Here, the light receivable region corresponds to the illumination region of the illumination light on the fundus Ef. For example, the illumination region on the fundus Ef can be identified based on the deflection angle of the deflection surface of the optical scanner 16. The main controller 61 can set the light receivable region on the light receiving surface of the image sensor 21, corresponding to the deflection direction of the deflection surface of the optical scanner 16 that is changed sequentially.

The illumination light that has been guided to the hole mirror 30 is reflected on the reflective surface on the peripheral part of the hole part, passes through the relay lens 40, is focused on the first focal point F1 of the first ellipsoidal concave mirror 51, is guided to the reflective surface of the first ellipsoidal concave mirror 51, and is reflected on this reflective surface. The illumination light reflected on the reflective surface of the first ellipsoidal concave mirror 51 is guided to the reflective surface of the second ellipsoidal concave mirror 52 via the second focal point F2 (first focal point F3) of the first ellipsoidal concave mirror 51. The illumination light guided to the reflective surface of the second ellipsoidal concave mirror 52 is reflected on this reflective surface, enters the eye of the eye E to be examined arranged at the second focal point F4 of the second ellipsoidal concave mirror 52, and is irradiated onto the fundus Ef.

The returning light from the eye E to be examined, which includes the returning light from the fundus Ef, travels in the same path as the outward path in the opposite direction, passes through the hole part formed in the hole mirror 30, passes through the focusing lens 22, and is received on the light receiving surface of the image sensor 21. On the light receiving surface of the image sensor 21, the light receivable region of the returning light corresponding to the illumination region of the illumination on the fundus Ef is set. Thereby, the returning light alone from fundus Ef can be received while suppressing the effect of unnecessary scattered light.

(S4: Terminated?)

Subsequently, the main controller 61 determines whether or not to terminate the scanning of the illumination light for the fundus Ef. For example, the main controller 61 can determine whether or not to terminate the scanning of the illumination light for the fundus Ef, by determining whether or not the deflection angle of the deflection surface of the hole mirror 30, which is changed sequentially, is within the predetermined deflection angular range.

When it is determined to terminate the scanning of the illumination light for the fundus Ef (S4: Y), the operation of the fundus imaging apparatus 1 proceeds to step S5. When it is determined not to terminate the scanning of the illumination light for the fundus Ef (S4: N), the operation of the fundus imaging apparatus 1 proceeds to step S3.

(S5: Acquire image)

When it is determined in step S4 to terminate the scanning of the illumination light for the fundus Ef (S4: Y), the main controller 61 controls the image forming unit 70 to form an image of the eye E to be examined based on the light receiving result(s) read out from the image sensor 21. In some embodiments, the image forming unit 70 sequentially forms the light receiving images based on the light receiving results read from the image sensor 21 in step S3, and forms an image of the eye E to be examined from the plurality of formed light receiving images.

This terminates the operation of the fundus imaging apparatus 1 (END).

As described above, according to the embodiments, the slit 14 is arranged so that the common plane of focal points including the four focal points of the two ellipsoidal concave mirrors is approximately parallel to the longitudinal direction of the slit image projected onto the fundus Ef, and the fundus Ef is scanned so that the slit image is moved in the direction orthogonal to the longitudinal direction of the slit image. Thereby, the contrast can be improved and the occurrence of the flare and/or ghost can be suppressed while securing a wide imaging angle of view at low cost with a simple configuration, without the need for additional correction optical systems. In addition, advanced optical design is not required.

Furthermore, by making the scan length variable, the scan range of the illumination light can be set as desired.

<First modification example>

The configuration of the fundus imaging apparatus according to the embodiments is not limited to the configuration of the fundus imaging apparatus 1 according to the embodiments. For example, the fundus imaging apparatus 1 according to the embodiments may further be provided with an OCT optical system.

Hereinafter, in the embodiments, a case of using the swept source type OCT method in the measurement or the imaging (shooting) using OCT will be described. However, the configuration according to the embodiments can also be applied to a fundus imaging apparatus using other type of OCT (for example, spectral domain type OCT).

In the following, the fundus imaging apparatus according to a first modification example of the embodiments will be described focusing on differences from the fundus imaging apparatus 1 according to the embodiments.

<Configuration>

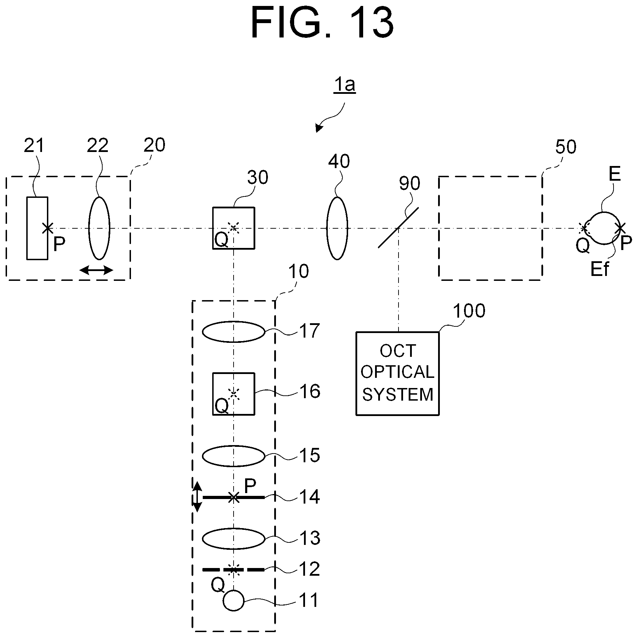

FIG. 13 shows an example of a configuration of an optical system of a fundus imaging apparatus according to the first modification example of the embodiments. In FIG. 13, like reference numerals designate like parts as in FIG. 1, and the redundant explanation may be omitted as appropriate.

The difference between the configuration of the optical system of the fundus imaging apparatus 1a according to the first modification example of the embodiments and the configuration of the optical system of the fundus imaging apparatus 1 according to the embodiments is that an OCT optical system 100 and a dichroic mirror 90 are added to the configuration of the optical system of the fundus imaging apparatus 1. The dichroic mirror 90 is arranged between the relay lens 40 and the relay optical system 50, as an optical path coupling separating member (optical path dividing member). In some embodiments, the dichroic mirror 90 is arranged between the hole mirror 30 and the relay lens 40.

The dichroic mirror 90 is an optical path coupling separating member that spatially separates the optical path of the illumination light and the optical path of the OCT optical system 100 (or that couples the optical path of the illumination light and the optical path of the OCT optical system 100). The dichroic mirror 90 reflects the measurement light from the OCT optical system 100 to guide the measurement light to the relay lens 40, and reflects the returning light of the measurement light from eye E to be examined to guide the returning light to the OCT optical system 100. Further, the dichroic mirror 90 transmits the illumination light that has been guided via the relay lens 40, and transmits the returning light from the eye E to be examined to guide the returning light to the relay lens 40.

(OCT optical system 100)

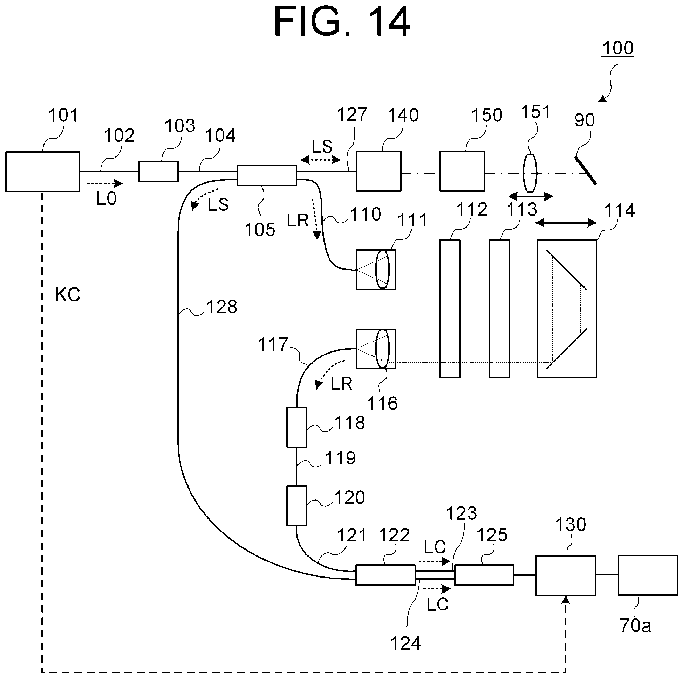

FIG. 14 shows an example of the configuration of the OCT optical system 100 in FIG. 13. In FIG. 14, parts similar to those in FIG. 13 are denoted by the same reference symbols, and description thereof is omitted as appropriate.

The OCT optical system 100 is provided with an optical system for performing OCT measurement (or OCT imaging) on the eye E to be examined. This optical system is an interference optical system that splits light from a wavelength sweeping type (i.e., a wavelength scanning type) light source into measurement light and reference light, makes the measurement light returning from the eye E to be examined and the reference light having traveled through the reference optical path interfere with each other to generate interference light, and detects the interference light. The detection result (detection signal) of the interference light obtained by the interference optical system is a signal indicating a spectrum of the interference light, and is sent to an image forming unit 70a, a data processor 75a, and the like which are described below.

Like swept source type fundus imaging apparatuses commonly used, the OCT light source 101 includes a wavelength sweeping type (i.e., a wavelength scanning type) light source capable of sweeping (scanning) the wavelengths of emitted light. A laser light source including a resonator and emitting light having a center wavelength of 1050 nm, for example, is used as the wavelength sweeping type light source. The OCT light source 101 temporally changes the output wavelength in the near infrared wavelength band which cannot be visually recognized by the human eye.

Light L0 output from the OCT light source 101 is guided to the polarization controller 103 through the optical fiber 102, and the polarized wave state of the light L0 is adjusted. The polarization controller 103, for example, applies external stress to the looped optical fiber 102 to thereby adjust the polarization state of the light L0 guided through the optical fiber 102.

The light L0 whose polarization state has been adjusted by the polarization controller 103 is guided to the fiber coupler 105 through the optical fiber 104, and is split into the measurement light LS and the reference light LR.

The reference light LR is guided to the collimator 111 through the optical fiber 110. The reference light LR is converted into a parallel light flux by the collimator 111. Then, the reference light LR is guided to the optical path length changing unit 114 via an optical path length correction member 112 and a dispersion compensation member 113. The optical path length correction member 112 acts so as to match the optical path length of the reference light LR with the optical path length of the measurement light LS. The dispersion compensation member 113 acts so as to match the dispersion characteristics between the reference light LR and the measurement light LS.

The optical path length changing unit 114 is movable in directions indicated by the arrow in FIG. 14, thereby changing the length of the optical path of the reference light LR. Through such movement, the length of the optical path of the reference light LR is changed. The change in the optical path length is used for the correction of the optical path length according to the axial length of the eye E to be examined, for the adjustment of the interference state, or the like. The optical path length changing unit 114 includes, for example, a corner cube and a movement mechanism for moving the corner cube. In this case, the corner cube in the optical path length changing unit 114 changes the traveling direction of the reference light LR that has been made into the parallel light flux by the collimator 111 in the opposite direction. The optical path of the reference light LR incident on the corner cube and the optical path of the reference light LR emitted from the corner cube are parallel.

The reference light LR that has traveled through the optical path length changing unit 114 passes through the dispersion compensation member 113 and the optical path length correction member 112, is converted from the parallel light flux to the convergent light flux by a collimator 116, and enters an optical fiber 117. The reference light LR that has entered the optical fiber 117 is guided to a polarization controller 118, and the polarization state of the reference light LR is adjusted. Then the reference light LR is guided to an attenuator 120 through an optical fiber 119, and the light amount of the reference light LR is adjusted. After that, the reference light LR is guided to a fiber coupler 122 through an optical fiber 121.

Meanwhile, the measurement light LS generated by the fiber coupler 105 is guided through the optical fiber 127, and is made into the parallel light flux by the collimator lens unit 140. The measurement light LS which has been made into the parallel light flux is deflected one-dimensionally or two-dimensionally using an optical scanner (optical scanner for OCT) 150.

The collimator lens unit 140 includes a collimator lens. The collimator lens is disposed on an optical axis of the interference optical system included in the OCT optical system 100. The collimator lens converts a light flux of the measurement light emitted from the end of an optical fiber into a parallel light flux. Here, the optical fiber is connected to the OCT optical system 100 and guides the measurement light LS to the end. The end of the optical fiber is arranged at the fundus conjugate position P, for example.

The optical scanner 150 (deflection surface) can be arranged at the iris conjugate position Q. In case of deflecting the illumination light in a one-dimensionally manner, the optical scanner 150 includes a galvano scanner that deflects the measurement light LS within a predetermined deflection angular range with reference to a predetermined deflection direction. In case of deflecting the illumination light in a two-dimensionally manner, the optical scanner 150 includes a first galvano scanner and a second galvano scanner. The first galvano scanner deflects the measurement light LS so as to move the irradiated position in a horizontal direction (for example, X direction) orthogonal to an optical axis of the OCT optical system 100. The second galvano scanner deflects the measurement light LS deflected by the first galvano scanner so as to move the irradiated position in a vertical direction (for example, Y direction) orthogonal to the optical axis of the OCT optical system 100. Examples of scan mode for moving the irradiated position of the measurement light LS using the optical scanner 150 include a horizontal scan, a vertical scan, a cross scan, a radial scan, a circle scan, a concentric scan, a helical (spiral) scan, and a Lissajous scan.

The measurement light LS deflected by the optical scanner 150 passes through a focusing lens 151, is reflected by the dichroic mirror 90 to guide to the reflective surface of the first ellipsoidal concave mirror 51, and is guided to the eye E to be examined along the same path as the illumination light from the illumination optical system 10. The focusing lens 151 is movable along the optical path of the measurement light LS (optical axis of the OCT optical system 100). The focusing lens 151 is moved along the optical path of the measurement light LS using a movement mechanism (not shown), under the control from the controller (controller 60a) described below.

The measurement light LS reflected on the reflective surface of the second ellipsoidal concave mirror 52 enters the eye E to be examined at the second focal point F4 (eye to be examined position) through the pupil. The measurement light LS is scattered (and reflected) at various depth positions of the eye E to be examined. The returning light of the measurement light LS including such backscattered light advances through the same path as the outward path in the opposite direction and is guided to the fiber coupler 105, and then reaches the fiber coupler 122 through the optical fiber 128.

The fiber coupler 122 combines (interferes) the measurement light LS incident through the optical fiber 128 and the reference light LR incident through the optical fiber 121 to generate interference light. The fiber coupler 122 generates a pair of interference light LC by splitting the interference light generated from the measurement light LS and the reference light LR at a predetermined splitting ratio (for example, 1 : 1). The pair of the interference light LC emitted from the fiber coupler 122 is guided to the detector 125 through the optical fibers 123 and 124, respectively.

The detector 125 is, for example, a balanced photodiode that includes a pair of photodetectors for respectively detecting the pair of interference light LC and outputs the difference between the pair of detection results obtained by the pair of photodetectors. The detector 125 sends the detection result(s) (i.e., interference signal(s)) to the DAQ (Data Acquisition System) 130. A clock KC is supplied from the OCT light source 101 to the DAQ 130. The clock KC is generated in the OCT light source 101 in synchronization with the output timing of each wavelength sweeping (scanning) within a predetermined wavelength range performed by the wavelength sweeping type light source. For example, the OCT light source 101 optically delays one of the two pieces of branched light obtained by branching the light L0 of each output wavelength, and then generates the clock KC based on the result of the detection of the combined light of the two pieces of branched light. The DAQ 130 performs sampling of the detection result obtained by the detector 125 based on the clock KC. The DAQ 130 sends the sampled detection result(s) obtained by the detector 125 to the image forming unit 70a, the data processor 75a, and the like. The image forming unit 70a (or data processor 75a) applies Fourier transform and the like to the spectral distribution based on the detection result(s) obtained by the detector 125, for example, with respect to a series of wavelength scans (for each A-line) to form the reflection intensity profile in each A-line. In addition, the image forming unit 70a forms image data by applying imaging processing to the reflection intensity profiles of the each A-line.

It should be noted that the difference between the optical path length of the measurement light and the optical path length of the reference light is changed by changing the optical path length of the reference light, in FIG. 14. However, the configuration according to the embodiments is not limited to this. For example, by changing the optical path length of the measurement light, the difference between the optical path length of the measurement light and the optical path length of the reference light may be configured to be changed.

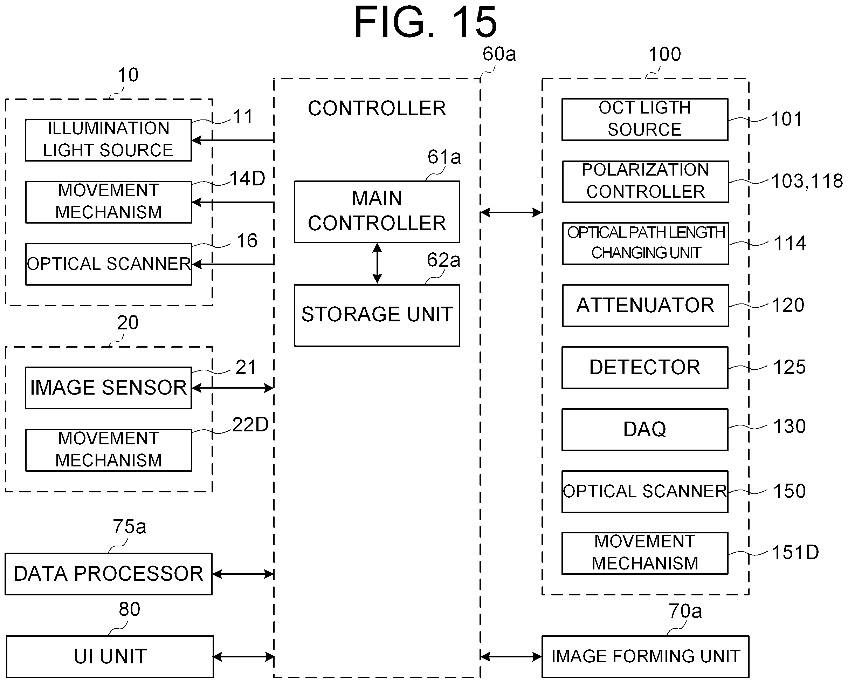

FIG. 15 shows an example of a configuration of a processing system of the fundus imaging apparatus 1a according to the first modification example of the embodiments. In FIG. 15, like reference numerals designate like parts as in FIG. 11, FIG. 13, or FIG. 14. The same description may not be repeated.

The differences between the configuration of the processing system of the fundus imaging apparatus 1a according to the first modification example and the configuration of the processing system of the fundus imaging apparatus 1 according to the embodiments are that the controller 60a is provided instead of the controller 60, that the image forming unit 70a is provided instead of the image forming unit 70, and that the data processor 75a and the OCT optical system 100 are added.

The controller 60a includes a main controller 61a and a storage unit 62a, and in addition to the controls that can be performed by the controller 60, the controller 60a performs control for the image forming unit 70a, the data processor 75a, and the OCT optical system 100. The functions of the main controller 61a are realized by a processor, for example, in the same way as the main controller 61. The storage unit 62a stores, in advance, computer programs for controlling the fundus imaging apparatus 1a, in the same way as the storage unit 62. Examples of the computer programs include an illumination light source control program, an image sensor control program, an optical scanner control program, an image forming program, a program for data processing, a program for controlling OCT optical system, a program for user interface. The main controller 61a operates according to the computer programs, and thereby the controller 60a performs the control processing.

The main controller 61a controls each of the illumination optical system 10, the imaging optical system 20, the image forming unit 70a, the data processor 75a, the OCT optical system 100, and the UI unit 80.

Examples of the control for the OCT optical system 100 include control for the OCT light source 101, operation control for the polarization controllers 103 and 118, movement control for the optical path length changing unit 114, operation control for the attenuator 120, control for the detector 125, control for the DAQ 130, control for the optical scanner 150, and control for a movement mechanism 151D.

Examples of the control for the OCT light source 101 include turning the light source on and off, adjustment of the light amount, adjustment of an aperture, and the like. Examples of the control for the detector 125 include adjustment of exposure of a detecting element, adjustment of gain of a detecting element, adjustment of detecting rate of a detecting element, and the like. Examples of the control for the optical scanner 150 include control of the scan direction, the scan position, the scan range, and scan speed by the optical scanner 150.

The movement mechanism 151D moves the focusing lens 151 in the optical axis direction of the OCT optical system 100. The main controller 61a outputs control signal(s) to the movement mechanism 151D to move the focusing lens 151 in a direction corresponding to the control signal(s) by the amount of movement corresponding to the control signal(s). For example, the fundus imaging apparatus 1 is provided with an actuator that generates a driving force for moving the movement mechanism 151D and a transmission mechanism that transmits the driving force from the actuator to the movement mechanism 151D. The actuator includes a pulse motor, for example. The transmission mechanism includes a combination of gears, a rack and pinion, and the like, for example. The movement mechanism 151D receives the driving force generated by the actuator controlled by the main controller 61a from the transmission mechanism, and moves the focusing lens 151 in the optical axis direction. Thereby, the focusing position of the measurement light LS is changed. The focusing position of the measurement light LS corresponds to the depth position (z position) of the beam waist of the measurement light LS.

Examples of the control for the image forming unit 70a include control of forming OCT images based on the detection result(s) of the interference light obtained by the OCT optical system 100, in addition to the image forming control that forms images of the eye E to be examined from the light receiving result(s) obtained using the image sensor 21.

Examples of the data processor 75a include control of image processing for the images formed by the image forming unit 70a and a control of analysis processing for images.

The image forming unit 70a can form the light receiving image (fundus image) corresponding to the arbitrary light receivable region based on the light receiving result(s) read out from the image sensor 21, in the same way as the image forming unit 70. The image forming unit 70a can sequentially form light receiving light images corresponding to the light receivable regions and form an image of the eye E to be examined from a plurality of the formed light receiving images.

Further, the image forming unit 70a forms image data of the OCT image (tomographic image) based on the detection signals input from the DAQ 130 (detector 125) and pixel position signals input from the controller 60a. Examples of the OCT image formed by the image forming unit 70a include an A-scan image and a B-scan image. The B-scan image is formed by arranging the A-scan images in the B-scan direction. As with the conventional swept source OCT, the image forming processing includes noise removal (noise reduction), filtering, dispersion compensation, fast Fourier transform (FFT), and the like. In the case of employing an OCT apparatus of another type, the image forming unit 70a performs known processing according to the type employed. The various images (the various image data) formed by the image forming unit 70a are stored in the storage unit 62a, for example.

The data processor 75a processes images formed based on the light receiving result(s) obtained by the imaging optical system 20 or data acquired by performing OCT measurement on the eye E to be examined. The data processor 75a can perform various kinds of image processing and various kinds of analysis processing on the image formed by the image forming unit 70a. For example, the data processor 75a performs various types of image correction processing such as brightness correction.

The data processor 75a performs known image processing such as interpolation processing for interpolating pixels between the OCT images to form image data of the three-dimensional image of the fundus Ef. It should be noted that the image data of the three-dimensional image means image data in which the positions of pixels are defined in a three-dimensional coordinate system. Examples of the image data of the three-dimensional image include image data defined by voxels three-dimensionally arranged. Such image data is referred to as volume data or voxel data. In case of displaying an image based on volume data, the data processor 75a performs image rendering processing (e.g., volume rendering, maximum intensity projection (MIP)) on the volume data to form image data of a pseudo three-dimensional image taken from a specific view direction. The pseudo three-dimensional image is displayed on a display device included in the UI unit 80.

Further, the three-dimensional image data may be stack data of a plurality of tomographic images. The stack data is image data formed by three-dimensionally arranging tomographic images along a plurality of scan lines based on positional relationship of the scan lines. That is, the stack data is image data obtained by representing tomographic images, which are originally defined in their respective two-dimensional coordinate systems, by a single three-dimensional coordinate system. That is, the stack data is image data formed by embedding tomographic images into a single three-dimensional space.

The data processor 75a can form a B-mode image (longitudinal cross-sectional image, axial cross-sectional image) in an arbitrary cross section, a C-mode image (transverse section image, horizontal cross-sectional image) in an arbitrary cross section, a projection image, a shadowgram, etc., by performing various renderings on the acquired three-dimensional data set (volume data, stack data, etc.). An image in an arbitrary cross section such as a B-mode image or a C-mode image is formed by selecting pixels (voxels) on a designated cross section from the three-dimensional data set. The projection image is formed by projecting the three-dimensional data set in a predetermined direction (Z direction, depth direction, axial direction). The shadowgram is formed by projecting a part of the three-dimensional data set (for example, partial data corresponding to a specific layer) in a predetermined direction. An image having a viewpoint on the front side of the eye to be examined, such as the C-mode image, the projection image, and the shadowgram, is called a front image (en-face image).

The data processor 75a can build (form) the B-mode image or the front image (blood vessel emphasized image, angiogram) in which retinal blood vessels and choroidal blood vessels are emphasized (highlighted), based on data (for example, B-scan image data) acquired in time series by OCT. For example, the OCT data in time series can be acquired by repeatedly scanning substantially the same site of the eye E to be examined.

In some embodiments, the data processor 75a compares the B-scan images in time series acquired by performing B-scan on substantially the same site, converts the pixel value of a change portion of the signal intensity into a pixel value corresponding to the change portion, and builds the emphasized image in which the change portion is emphasized. Further, the data processor 75a forms an OCTA image by extracting information of a predetermined thickness at a desired site from a plurality of built emphasized images and building as an en-face image.

An image (for example, a three-dimensional image, a B-mode image, a C-mode image, a projection image, a shadowgram, and an OCTA image) generated by the data processor 75a is also included in the OCT image.

Further, the data processor 75a performs predetermined analysis processing on the image formed based on the light receiving result(s) obtained by the imaging optical system 20, the detection result(s) of the interference light acquired by the OCT measurement, or the OCT image formed based on the detection result(s). Examples of the predetermined analysis processing include identification of a predetermined site (tissue, lesion) of the eye E to be examined; calculation of a distance, area, angle, ratio, or density between designated sites (distance between layers, interlayer distance); calculation by a designated formula; identifying of the shape of a predetermined site; calculation of these statistics; calculation of distribution of the measured value or the statistics; image processing based on these analysis processing results, and the like. Examples of the predetermined tissue include a blood vessel, an optic disc, a fovea, a macula, and the like. Examples of the predetermined lesion include a leukoma, a hemorrhage, and the like.

The fundus imaging apparatus 1a may include a movement mechanism that moves the OCT optical system 100 in a one-dimensional direction or two-dimensional directions, which intersect(s) the optical axis of the OCT optical system 100. In this case, the main controller 61a controls this movement mechanism to move the OCT optical system 100 relative to the dichroic mirror 90 in the one-dimensional direction or two-dimensional directions, which intersect(s) the optical axis of the OCT optical system 100. This allows to move the scan range using the optical scanner 150 in the OCT optical system 100 and to scan the wide-angle scan range on the fundus Ef.

The dichroic mirror 90 is an example of the "optical path coupling separating member" according to the embodiments.

<Operation>

Next, the operation of the fundus imaging apparatus 1a according to the first modification example of the embodiments will be described.

The fundus imaging apparatus 1a can perform OCT measurement using the OCT optical system 100 in parallel with the scan control for the fundus Ef using the slit-shaped illumination light from the illumination optical system 10. In some embodiments, the fundus imaging apparatus 1a performs OCT measurement using the OCT optical system 100 in a state where the scan control for the fundus Ef using the slit-shaped illumination light from the illumination optical system 10 is stopped. Hereafter, the control of OCT measurement that can be performed in parallel with the scan control for the fundus Ef using the slit-shaped illumination light will be described.

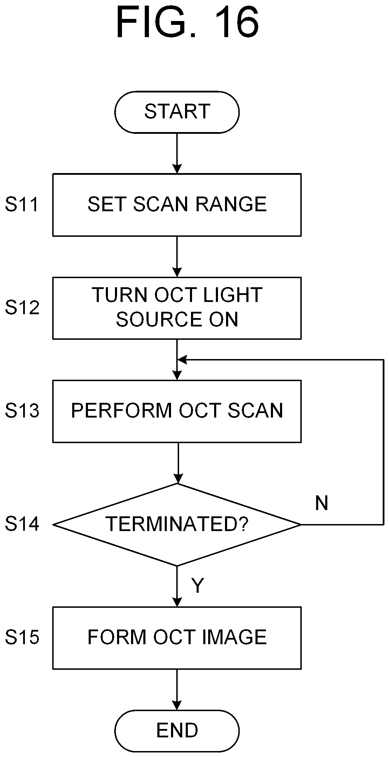

FIG. 16 shows an example of an operation of the fundus imaging apparatus 1a according to the first modification example of the embodiments. FIG. 16 represents a flowchart of the example of the operation of the fundus imaging apparatus 1a according to the first modification example. The storage unit 62a stores computer program(s) for realizing the processing shown in FIG. 16. The main controller 61a operates according to the computer programs, and thereby the main controller 61a performs the processing shown in FIG. 16.

It is assumed that the eye E to be examined is arranged at a predetermined eye to be examined position (the second focal point F4 of the second ellipsoidal concave mirror 52), in FIG. 16.

(S11: Set scan range)

First, the main controller 61a sets the scan range of the optical scanner 150. The main controller 61a can set the scan start position, the scan end position, the scan speed (scan frequency), etc. by the optical scanner 150, along with the scan range.

In some embodiments, the user can designate the scan mode or the operation mode by operating the operation device in the UI unit 80. When the scan mode (for example, horizontal scan, vertical scan) is designated by operating the operation device by the user, the main controller 61a analyzes operation information from the operation device to identify the designated scan mode. When the operation mode is designated by operating the operation device by the user, the main controller 61a analyzes the operation information to identify a scan mode (for example, horizontal scan, vertical scan) designated in advance in the designated operation mode (for example, OCT measurement mode).

(S12: Turn OCT light source on)

Subsequently, the main controller 61a controls the OCT light source 101 to turn the OCT light source 101 on. In some embodiments, the main controller 61a performs step S12 in synchronization with the turning on control of the illumination light source 11 in step S2 shown in FIG. 12.

In some embodiments, the main controller 61a performs controls of adjusting focusing and of adjusting polarization. For example, the main controller 61a controls the OCT optical system 100 to perform OCT measurement, after controlling the movement mechanism 151D to move the focusing lens by a predetermined distance. The main controller 61a controls the data processor 75a to determine the focus state of the measurement light LS based on the detection result of the interference light acquired by the OCT measurement. For example, the data processor 75a analyzes the detection results of the interference light acquired by the OCT measurements to calculate predetermined evaluation value(s) relating to the image quality of OCT images and to determine the focus state based on the calculated evaluation value(s). When it is determined that the focus state is not appropriate based on the determination result of the data processor 75a, the main controller 61a controls a movement mechanism 151D again and repeats this until it is determined that the focus state of the measurement light LS is appropriate.

Further, for example, the main controller 61a controls the OCT optical system 100 to perform OCT measurement after controlling at least one of the polarization controller 103 or the polarization controller 118 to change the polarization state of at least one of the light L0 or the measurement light LS by a predetermined amount. And then, the main controller 61a controls the image forming unit 70a to form the OCT image on the basis of the acquired detection result of the interference light. The main controller 61a controls the data processor 75a to determine the image quality of the OCT image acquired by performing the OCT measurement. When it is determined that the polarization state of the measurement light LS is not appropriate based on the determination result of the data processor 75a, the main controller 61a controls the polarization controllers 103 and 118 again and repeats this until it is determined that the polarization state of the measurement light LS is appropriate.

(S13: Perform OCT scan)

Subsequently, the main controller 61a controls the optical scanner 150 to deflect the measurement light LS generated based on the light L0 emitted from the OCT light source 101 to scan a predetermined site on the fundus Ef of the eye E to be examined with the deflected measurement light LS. The detection result of the interference light acquired by the OCT measurement is sampled by the DAQ 130 and is stored as the interference signal in the storage unit 62a or the like.

(S14: Terminated?)

Subsequently, the main controller 61a determines whether or not to terminate the OCT scan for the fundus Ef. For example, the main controller 61a can determine whether or not to terminate the OCT scan for the fundus Ef, by determining whether or not the deflection angle of the deflection surface of the optical scanner 150, which is changed sequentially, is within the predetermined deflection angular range.

When it is determined to terminate the OCT scan for the fundus Ef (S14: Y), the operation of the fundus imaging apparatus 1a proceeds to step S15. When it is determined not to terminate the OCT scan for the fundus Ef (S14: N), the operation of the fundus imaging apparatus 1a proceeds to step S13.

(S15: Form OCT image)

When it is determined in step S14 to terminate the OCT scan for the fundus Ef (S14: Y), the main controller 61a controls the image forming unit 70a to form a plurality of A-scan images of the fundus Ef along the B-scan direction, based on the interference signal(s) acquired by performing OCT scan in step S13. In some embodiments, the main controller 61a controls the data processor 75a to form OCT images such as three-dimensional OCT images, B-mode images, C-mode images, projection images, shadowgrams, and OCTA images.

This terminates the operation of the fundus imaging apparatus 1a (END).

As described above, according to the first modification example of the embodiments, in addition to the effects obtained in the embodiments, OCT measurement (OCT imaging) can be performed on any position on the fundus being observed at a large wide angle, without sharing an optical scanner for OCT scanning and an optical scanner for deflection of illumination light.

<Second modification example>

The configuration of the fundus imaging apparatus according to the embodiments is not limited to the configuration according to the embodiments or the first modification example thereof described above. For example, in the configuration shown in FIG. 2 and FIG. 3, three or more curved mirrors (aspheric mirrors) may be provided instead of the first ellipsoidal concave mirror 51 and the second ellipsoidal concave mirror 52.

In a second modification example, two concave mirrors and a single convex mirror are provided instead of the first ellipsoidal concave mirror 51 and the second ellipsoidal concave mirror 52.

In the following, the fundus imaging apparatus according to the second modification example of the embodiments will be described focusing on differences from the fundus imaging apparatus 1 according to the embodiments.

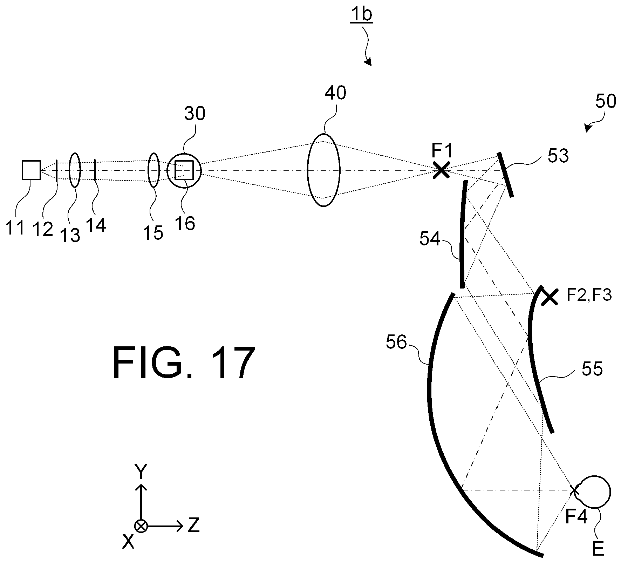

FIG. 17 shows an example of a configuration of an optical system of a fundus imaging apparatus 1b according to the second modification example of the embodiments. In FIG. 17, like reference numerals designate like parts as in FIG. 3, and the redundant explanation may be omitted as appropriate.

The difference between the configuration of the fundus imaging apparatus 1b according to the second modification example and the configuration of the fundus imaging apparatus 1 according to the embodiments is that a reflective mirror 53 as a plane mirror, a hyperboloidal concave mirror 54 as a concave mirror, a hyperboloidal convex mirror 55 as a convex mirror, and an ellipsoidal concave mirror 56 as a concave mirror are provided instead of the first ellipsoidal concave mirror 51 and the second ellipsoidal concave mirror 52.

The reflective mirror 53 reflects the slit-shaped illumination light from the illumination optical system 10 to guide to the hyperboloidal concave mirror 54. Further, the reflective mirror 53 guides the returning light from the eye E to be examined, which is reflected by the hyperboloidal concave mirror 54, to the relay lens 40. In some embodiments, the fundus imaging apparatus 1b may have a configuration in which the reflective mirror 53 is omitted.

By deflecting the optical axis using the reflective mirror 53, the size in the depth direction (z direction) of the optical system of the fundus imaging apparatus 1b can be reduced.

(Hyperboloidal concave mirror 54)

The hyperboloidal concave mirror 54 has a concave-shaped reflective surface. The reflective surface of the hyperboloidal concave mirror 54A is a hyperboloid. The hyperboloidal concave mirror 54 is an example of the concave mirror. One of focal points of the hyperboloidal concave mirror 54 is the first focal point F1.

(Hyperboloidal convex mirror 55)

The hyperboloidal convex mirror 55 has a convex-shaped reflective surface. A reflective surface of the hyperboloidal convex mirror 55 is a hyperboloid. The hyperboloidal convex mirror 55 is an example of the convex mirror. One of focal points of the hyperboloidal convex mirror 55 is the second focal point F2.

(ellipsoidal concave mirror 56)

A reflective surface of the ellipsoidal concave mirror 56 is a concave-shaped elliptical surface. The ellipsoidal concave mirror 56 is an example of the concave mirror.

The ellipsoidal concave mirror 56 has two optically conjugate focal points (first focal point F3, second focal point F4). The ellipsoidal concave mirror 56 is arranged so that the first focal point F3 substantially coincides with the second focal point F2 of the hyperboloidal convex mirror 55. In some embodiments, the ellipsoidal concave mirror 56 is arranged so that the first focal point F3 substantially coincides with a position conjugate optically to the second focal point F2 of the hyperboloidal convex mirror 55 (conjugate position of the second focal point F2) or near the position. The eye E to be examined is arranged at the second focal point F4 of the ellipsoidal concave mirror 56. In other words, the ellipsoidal concave mirror 56 is positioned so that the second focal point F4 substantially coincides with eye to be examined position where the eye E to be examined is positioned.

In such a configuration, the slit-shaped illumination light transmitted through the relay lens 40 is focused on the first focal point F1, is reflected on the reflective surface of the reflective mirror 53, is reflected on the concave-shaped reflective surface of the hyperboloidal concave mirror 54, is reflected on the convex-shaped reflective surface of the hyperboloidal convex mirror 55, is reflected on the reflective surface of the ellipsoidal concave mirror 56, and is guided to the eye E to be examined arranged at the second focal point F4 of the ellipsoidal concave mirror 56.

The illumination light that has been guided to the eye E to be examined enters the eye through the pupil and is irradiated onto the fundus Ef. The returning light from the fundus Ef (eye E to be examined) is emitted outside of the eye E to be examined through the pupil, travels in the same path as the outward path in the opposite direction, and is guided to the first focal point F1 of the hyperboloidal concave mirror 54. The returning light that has been guided to the first focal point F1 is transmitted through the relay lens 40, passes through the hole part formed in the hole mirror 30, and is guided to the imaging optical system 20, as described above.

In some embodiments, at least one of the hyperboloidal concave mirror 54 or the hyperboloidal convex mirror 55 is a paraboloidal mirror. In some embodiments, at least one of the hyperboloidal concave mirror 54, the hyperboloidal convex mirror 55, or the ellipsoidal concave mirror 56 is formed so that the reflective surface is a free-form surface.

[Actions]

The fundus imaging apparatus according to the embodiments or the modification examples thereof will be described.

The first aspect of the embodiments is a fundus imaging apparatus (1, 1a, 1b) including two or more curved mirrors (first ellipsoidal concave mirror 51 and second ellipsoidal concave mirror 52, or reflective mirror 53, hyperboloidal concave mirror 54, hyperboloidal convex mirror 55, and ellipsoidal concave mirror 56), an illumination optical system (10), an image sensor (21), and a controller (60 (main controller 61), 60a (main controller 61a)). The illumination optical system is configured to irradiate slit-shaped illumination light onto a fundus (Ef) of an eye (E) to be examined through the two or more curved mirrors, and to illuminate the fundus while moving an illumination region. The image sensor can be arranged at a fundus conjugate position (P) substantially conjugate optically to the fundus, and is configured to receive returning light from the eye to be examined. The controller is configured to control at least the illumination optical system. A longitudinal direction of an image (slit image) formed by the illumination light on the fundus is approximately parallel to a plane (common plane PL of focal points) including two or more focal points (first focal point F1, second focal point F2, first focal point F3, and second focal point F4) of the two or more curved mirrors. The controller is configured to control the illumination optical system so that the image described above moves in a direction intersecting (orthogonal to) the longitudinal direction on the fundus.

According to such an aspect, the separation between the slit image on the fundus and the light receivable region (light-receptive region) of the returning light at the fundus conjugate position substantially conjugate optically to the fundus can be minimized, without being affected by aberrations of the two or more curved mirrors that relay the slit-shaped illumination light. As a result, the contrast of the image can be improved and the occurrence of flare and/or ghost can be reduced at low cost with a simple configuration, without the need for advanced optical design. Therefore, higher-quality fundus images of the eye to be examined can be acquired.

In the second aspect of the embodiments, in the first embodiment, the illumination optical system includes an optical scanner (16) that can be arranged at an iris conjugate position (Q) substantially conjugate optically to an iris of the eye to be examined, and is configured to illuminate the fundus with the illumination light in a movable manner by deflecting the illumination light using the optical scanner.

According to such an aspect, the high-quality image of the fundus of the eye to be examined can be acquired with a simple configuration, using the deflection control of the illumination light using the optical scanner.

In the third aspect of the embodiments, in the first aspect or the second aspect, the illumination optical system includes: a light source (illumination light source 11); and a slit (14) that can be arranged at the fundus conjugate position. The illumination optical system is configured to generate the slit-shaped illumination light by irradiating light from the light source onto the slit.

According to such an aspect, the slit-shaped illumination can be generated and the high-quality image of the fundus of the eye to be examined can be acquired, with a simple configuration.

In the fourth aspect of the embodiments, in the third aspect, the slit is configured to be capable of moving in an optical axis direction of the illumination optical system in accordance with a dioptric power (refractive power) of the eye to be examined.

According to such an aspect, the fundus can be illuminated with high-intensity illumination light regardless of the dioptric power of the eye to be examined.

The fifth aspect of the embodiments, in any of the first aspect to the fourth aspect, further includes an iris aperture (12) in that an opening (aperture) is formed at an eccentric position from an optical axis, and that can be arranged at an iris conjugate position (Q) substantially conjugate optically to an iris of the eye to be examined, and an optical path dividing member (hole mirror 30) arranged between the two or more curved mirrors and the image sensor, and configured to spatially divide an optical path of the illumination light and an optical path of the returning light.

According to such an aspect, the pupil dividing between the illumination light incident within the eye and the returning light emitted from inside the eye to the outside can be performed, with a simple configuration. Thereby, higher-quality images of the fundus can be acquired.

The sixth aspect of the embodiments, in the fifth aspect, further includes a focusing lens (22) arranged between the image sensor and the optical path dividing member, and configured to be capable of moving in an optical axis direction.

According to such an aspect, the returning light from the eye to be examined can be imaged on the light receiving surface of the image sensor, regardless of the dioptric power (refractive power) of the eye to be examined. Thereby, higher-quality images of the fundus can be acquired.

In the seventh aspect of the embodiments, in any one of the first aspect to the sixth aspect, the two or more curved mirrors are arranged so as to share at least one of the two or more focal points.

According to such an aspect, the image formed by the illumination light can be relayed with high accuracy with a simple configuration. Thereby, higher-quality images of the fundus can be acquired.

In the eighth aspect of the embodiments, in any one of the first aspect to the seventh aspect, the two curved mirrors include an ellipsoidal concave mirror.

According to such an aspect, higher-quality images of the fundus can be acquired, using the ellipsoidal concave mirror that can be manufactured with high precision.

In the ninth aspect of the embodiments, in any one of the first aspect to the eighth aspect, the two or more curved mirrors are a first ellipsoidal concave mirror (51) and a second ellipsoidal concave mirror (52). A first focal point (F1) of the first ellipsoidal concave mirror is arranged at an iris conjugate position substantially conjugate optically to an iris of the eye to be examined. A second focal point (F2) of the first ellipsoidal concave mirror approximately coincides with a first focal point (F3) of the second ellipsoidal concave mirror, and a second focal point (F4) of the second ellipsoidal concave mirror can be arranged at the iris.

According to such an aspect, wide-angle images of the fundus can be acquired with higher image quality, using the two ellipsoidal concave mirrors.

In the tenth aspect of the embodiments, in any one of the first aspect to the ninth aspect, the image sensor is an image sensor using a rolling shutter system.

According to such an aspect, high-quality images of the fundus can be acquired with a simple configuration.

The eleventh aspect of the embodiments is a method of controlling a fundus imaging apparatus (1, 1a, 1b) including: two or more curved mirrors (first ellipsoidal concave mirror 51 and second ellipsoidal concave mirror 52, or reflective mirror 53, hyperboloidal concave mirror 54, hyperboloidal convex mirror 55, and ellipsoidal concave mirror 56); an illumination optical system (10); and an image sensor (21). The illumination optical system is configured to irradiate slit-shaped illumination light onto a fundus (Ef) of an eye (E) to be examined through the two or more curved mirrors, and to illuminate the fundus while moving an illumination region. The image sensor can be arranged at a fundus conjugate position substantially conjugate optically to the fundus, and is configured to receive returning light from the eye to be examined. A longitudinal direction of an image (slit image) formed by the illumination light on the fundus is approximately parallel to a plane (common plane PL of focal points) including two or more focal points of the two or more curved mirrors. The method of controlling the fundus imaging apparatus includes a control step and a capturing step. The control step is performed to control the illumination optical system so that the image described above moves in a direction intersecting the longitudinal direction described above on the fundus. The capturing step is performed to capture light receiving result of the returning light in a light-receptive region (light receivable region) on a light receiving surface (SR) of the image sensor, the light-receptive region corresponding to a position of the image described above on the fundus.

According to such a method, the separation between the slit image on the fundus and the light receivable region (light-receptive region) of the returning light at the fundus conjugate position substantially conjugate optically to the fundus can be minimized, without being affected by aberrations of the two or more curved mirrors that relay the slit-shaped illumination light. As a result, the contrast of the image can be improved and the occurrence of flare and/or ghost can be reduced at low cost with a simple configuration, without the need for advanced optical design. Therefore, higher-quality fundus images of the eye to be examined can be acquired.

In the twelfth aspect of the embodiments, in the eleventh aspect, the illumination optical system includes: a light source (illumination light source 11); and a slit (14) that can be arranged at the fundus conjugate position. The method of controlling the fundus imaging apparatus includes a a slit control step of moving in an optical axis direction of the illumination optical system in accordance with a dioptric power of the eye to be examined.

According to such a method, the fundus can be illuminated with high-intensity illumination light regardless of the dioptric power of the eye to be examined.

In the thirteenth aspect of the embodiments, in the eleventh aspect or the twelfth aspect, the fundus imaging apparatus further includes: an iris aperture (12) in that an opening is formed at an eccentric position from an optical axis, and that can be arranged at an iris conjugate position (Q) substantially conjugate optically to an iris of the eye to be examined; an optical path dividing member (hole mirror 30) arranged between the two or more curved mirrors and the image sensor, and configured to spatially couple an optical path of the illumination light and an optical path of the returning light. The method of controlling the fundus imaging apparatus further includes a focus control step of moving the focusing lens in an optical axis direction.

According to such a method, the returning light from the eye to be examined can be imaged on the light receiving surface of the image sensor, regardless of the dioptric power (refractive power) of the eye to be examined. Thereby, higher-quality images of the fundus can be acquired.

In the fourteenth aspect of the embodiments, in any one of the eleventh aspect to the thirteenth aspect, the capturing step is performed to capture the light receiving result of the returning light using a rolling shutter system.

According to such a method, high-quality images of the fundus can be acquired with a simple control.

In the fifteenth aspect of the embodiments, in any one of the eleventh aspect to the fourteenth aspect, the two or more curved mirrors are a first ellipsoidal concave mirror (51) and a second ellipsoidal concave mirror (52). A first focal point (F1) of the first ellipsoidal concave mirror is arranged at an iris conjugate position substantially conjugate optically to an iris of the eye to be examined. A second focal point (F2) of the first ellipsoidal concave mirror approximately coincides with a first focal point (F3) of the second ellipsoidal concave mirror, and a second focal point (F4) of the second ellipsoidal concave mirror can be arranged at the iris.

According to such an aspect, wide-angle images of the fundus can be acquired with higher image quality, using the two ellipsoidal concave mirrors.

The sixteenth aspect of the embodiments is a program of causing a computer to execute each step of the method of controlling the fundus imaging apparatus of any one of the eleventh aspect to the fifteenth aspect.

According to such a program, the separation between the slit image on the fundus and the light receivable region (light-receptive region) of the returning light at the fundus conjugate position substantially conjugate optically to the fundus can be minimized, without being affected by aberrations of the two or more curved mirrors that relay the slit-shaped illumination light. As a result, the contrast of the image can be improved and the occurrence of flare and/or ghost can be reduced at low cost with a simple configuration, without the need for advanced optical design. Therefore, higher-quality fundus images of the eye to be examined can be acquired.

< Others >

The above-described embodiments are merely examples for carrying out the present invention. Those who intend to implement the present invention can apply any modification, omission, addition, or the like within the scope of the gist of the present invention.

In some embodiments, a program for causing a processor (computer) to execute the method of controlling the fundus imaging apparatus described above is provided. Such a program can be stored in any non-transitory recording medium (storage medium) that can be read by a computer. Examples of the recording medium include a semiconductor memory, an optical disk, a magneto-optical disk (CD-ROM, DVD-RAM, DVD-ROM, MO, etc.), a magnetic storage medium (hard disk, floppy (registered trade mark) disk, ZIP, etc.), and the like. The computer program may be transmitted and received through a network such as the Internet, LAN, etc.

[EXPLANATION OF SYMBOLS]

1, 1a, 1b Fundus imaging apparatus
10 Illumination optical system
11 Illumination light source
12 Iris aperture
14 Slit
16 Optical scanner
20 Imaging optical system
21 Image sensor
22 Focusing lens
30 Hole mirror
40 Relay lens
50 Relay optical system
51 First ellipsoidal concave mirror
52 Second ellipsoidal concave mirror
60, 60a Controller
61, 61a Main controller
62, 62a Storage unit
70, 70a Image forming unit
75a Data processor
80 UI unit
90 Dichroic mirror
100 OCT optical system
E Eye to be examined
Ef Fundus
F1, F3 First focal point
F2, F4 Second focal point
P Fundus conjugate position
Q Iris conjugate position

**Claims**

1. A fundus imaging apparatus, comprising:

   two or more curved mirrors;
   an illumination optical system configured to irradiate slit-shaped illumination light onto a fundus of an eye to be examined through the two or more curved mirrors, and to illuminate the fundus while moving an illumination region;
   an image sensor that is arrangeable at a fundus conjugate position substantially conjugate optically to the fundus, and configured to receive returning light from the eye to be examined; and
   a controller configured to control at least the illumination optical system, wherein

   a longitudinal direction of an image formed by the illumination light on the fundus is approximately parallel to a plane including two or more focal points of the two or more curved mirrors, and
   the controller is configured to control the illumination optical system so that the image moves in a direction intersecting the longitudinal direction on the fundus.

**2.** The fundus imaging apparatus of claim 1, wherein
the illumination optical system includes an optical scanner arrangeable at an iris conjugate position substantially conjugate optically to an iris of the eye to be examined, and is configured to movably illuminate the fundus with the illumination light by deflecting the illumination light using the optical scanner.

**3.** The fundus imaging apparatus of claim 1, wherein
the illumination optical system includes:

   a light source; and
   a slit arrangeable at the fundus conjugate position, wherein
   the illumination optical system is configured to generate the slit-shaped illumination light by irradiating light from the light source onto the slit.

**4.** The fundus imaging apparatus of claim 3, wherein
the slit is configured to be movable in an optical axis direction of the illumination optical system in accordance with a dioptric power of the eye to be examined.

**5.** The fundus imaging apparatus of claim 3, further comprising:

   an iris aperture in that an opening is formed at an eccentric position from an optical axis, and that is arrangeable at an iris conjugate position substantially conjugate optically to an iris of the eye to be examined; and
   an optical path dividing member arranged between the two or more curved mirrors and the image sensor, and configured to spatially divide an optical path of the illumination light and an optical path of the returning light.

**6.** The fundus imaging apparatus of claim 5, further comprising:
a focusing lens arranged between the image sensor and the optical path dividing member, and configured to be movable in an optical axis direction.

**7.** The fundus imaging apparatus of any one of claims 1 to 6, wherein
the two or more curved mirrors are arranged so as to share at least one of the two or more focal points.

**8.** The fundus imaging apparatus of any one of claims 1 to 6, wherein
the two curved mirrors include an ellipsoidal concave mirror.

**9.** The fundus imaging apparatus of any one of claims 1 to 6, wherein

   the two or more curved mirrors are a first ellipsoidal concave mirror and a second ellipsoidal concave mirror,
   a first focal point of the first ellipsoidal concave mirror is arranged at an iris conjugate position substantially conjugate optically to an iris of the eye to be examined,
   a second focal point of the first ellipsoidal concave mirror approximately coincides with a third focal point of the second ellipsoidal concave mirror, and
   a fourth focal point of the second ellipsoidal concave mirror is arrangeable at the iris.

**10.** The fundus imaging apparatus of any one of claims 1 to 6, wherein
the image sensor is an image sensor using a rolling shutter system.

**11.** A method of controlling a fundus imaging apparatus comprising:

   two or more curved mirrors;
   an illumination optical system configured to irradiate slit-shaped illumination light onto a fundus of an eye to be examined through the two or more curved mirrors, and to illuminate the fundus while moving an illumination region; and
   an image sensor that is arrangeable at a fundus conjugate position substantially conjugate optically to the fundus, and configured to receive returning light from the eye to be examined, wherein
   a longitudinal direction of an image formed by the illumination light on the fundus is approximately parallel to a plane including two or more focal points of the two or more curved mirrors, the method comprising:

      a control step of controlling the illumination optical system so that the image moves in a direction intersecting the longitudinal direction on the fundus; and

a capturing step of capturing light receiving result of the returning light in a light-receptive region on a light receiving surface of the image sensor, the light-receptive region corresponding to a position of the image on the fundus.

12. The method of controlling the fundus imaging apparatus of claim 11, wherein
the illumination optical system includes:

a light source; and
a slit arrangeable at the fundus conjugate position, and
the method further comprises
a slit control step of moving in an optical axis direction of the illumination optical system in accordance with a dioptric power of the eye to be examined.

13. The method of controlling the fundus imaging apparatus of claim 11, wherein
the fundus imaging apparatus further comprises:

an iris aperture in that an opening is formed at an eccentric position from an optical axis, and that is arrangeable at an iris conjugate position substantially conjugate optically to an iris of the eye to be examined;
an optical path dividing member arranged between the two or more curved mirrors and the image sensor, and configured to spatially couple an optical path of the illumination light and an optical path of the returning light; and
a focusing lens arranged between the image sensor and the optical path dividing member, and
the method further comprises
a focus control step of moving the focusing lens in an optical axis direction.

14. The method of controlling the fundus imaging apparatus of any one of claims 11 to 13, wherein
the capturing step is performed to capture the light receiving result of the returning light using a rolling shutter system.

15. The method of controlling the fundus imaging apparatus of any one of claims 11 to 13, wherein

the two or more curved mirrors are a first ellipsoidal concave mirror and a second ellipsoidal concave mirror,
a first focal point of the first ellipsoidal concave mirror is arranged at an iris conjugate position substantially conjugate optically to an iris of the eye to be examined,
a second focal point of the first concave mirror approximately coincides with a third focal point of the second ellipsoidal concave mirror, and
a fourth focal point of the second ellipsoidal concave mirror is arrangeable at the iris.

16. A program of causing a computer to execute each step of the method of controlling the fundus imaging apparatus of any one of claims 11 to 13.

# FIG. 1

FIG. 2

EP 4 555 919 A1

50

51

40

30

F1

11 12 13 14 15 16

F2,F3

FIG. 3

E

d
d'

52

F4

Y

X Z

# FIG. 4

# FIG. 5

# FIG. 6

# FIG. 7

# FIG. 8

# FIG. 9A

X

c ⟷ c′

Y ↕

SL
14

# FIG. 9B

X

b ⟷ b′

Y ↕

LA

SR

# FIG. 9C

LA0    Ef

d

d′

SL0

# FIG. 10A

Side View

Top View

# FIG. 10B

Side View

Top View

# FIG. 11

10

ILLUMINATION LIGHT SOURCE — 11

MOVEMENT MECHANISM — 14D

OPTICAL SCANNER — 16

20

IMAGE SENSOR — 21

MOVEMENT MECHANISM — 22D

60

CONTROLLER

MAIN CONTROLLER — 61

STORAGE UNIT — 62

70

IMAGE FORMING UNIT

80

UI UNIT

# FIG. 12

START

S1 — PERFORM FOCUSING

S2 — TURN ILLUMINATION LIGHT SOURCE ON

S3 — PERFORM DEFLECTION CONTROL OF ILLUMINATION LIGHT / SET LIGHT RECEIVABLE REGION OF LIGHT RECEIVING SURFACE

S4 — TERMINATED? — N

Y

S5 — ACQUIRE IMAGE

END

# FIG. 13

# FIG. 14

# FIG. 15

CONTROLLER 60a

**10**
- ILLUMINATION LIGHT SOURCE — 11
- MOVEMENT MECHANISM — 14D
- OPTICAL SCANNER — 16

**20**
- IMAGE SENSOR — 21
- MOVEMENT MECHANISM — 22D

MAIN CONTROLLER — 61a

STORAGE UNIT — 62a

DATA PROCESSOR — 75a

UI UNIT — 80

**100**
- OCT LIGTH SOURCE — 101
- POLARIZATION CONTROLLER — 103, 118
- OPTICAL PATH LENGTH CHANGING UNIT — 114
- ATTENUATOR — 120
- DETECTOR — 125
- DAQ — 130
- OPTICAL SCANNER — 150
- MOVEMENT MECHANISM — 151D

IMAGE FORMING UNIT — 70a

EP 4 555 919 A1

# FIG. 16

```
                    ┌──────────┐
                    │  START   │
                    └────┬─────┘
                         │
                         ▼
        ┌──────────────────────────────┐
   S11  │       SET SCAN RANGE         │
        └───────────────┬──────────────┘
                        │
                        ▼
        ┌──────────────────────────────┐
   S12  │       TURN OCT LIGHT         │
        │        SOURCE ON             │
        └───────────────┬──────────────┘
                        │◄───────────────────┐
                        ▼                    │
        ┌──────────────────────────────┐    │
   S13  │      PERFORM OCT SCAN        │    │
        └───────────────┬──────────────┘    │
                        │                    │
                        ▼                    │
   S14       ◄ TERMINATED? ►    N ───────────┘
                        │
                        │ Y
                        ▼
        ┌──────────────────────────────┐
   S15  │      FORM OCT IMAGE          │
        └───────────────┬──────────────┘
                        │
                        ▼
                    ┌──────────┐
                    │   END    │
                    └──────────┘
```

FIG. 17

# EP 4 555 919 A1

## INTERNATIONAL SEARCH REPORT

International application No.

**PCT/JP2023/031792**

| A. | CLASSIFICATION OF SUBJECT MATTER |
|---|---|

*A61B 3/12*(2006.01)i; *A61B 3/14*(2006.01)i
FI:   A61B3/12; A61B3/14

According to International Patent Classification (IPC) or to both national classification and IPC

| B. | FIELDS SEARCHED |
|---|---|

Minimum documentation searched (classification system followed by classification symbols)

A61B3/00-3/18

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | WO 2022/124170 A1 (TOPCON CORP.) 16 June 2022 (2022-06-16)<br>paragraphs [0040]-[0101], fig. 1-3 | 1-16 |
| A | JP 2018-167000 A (NIDEK CO., LTD.) 01 November 2018 (2018-11-01)<br>entire text, all drawings | 1-16 |
| A | WO 2018/088338 A1 (KOWA COMPANY, LTD.) 17 May 2018 (2018-05-17)<br>entire text, all drawings | 1-16 |

☐ Further documents are listed in the continuation of Box C.     ☑ See patent family annex.

| | |
|---|---|
| *      Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **25 September 2023** | **10 October 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/031792**

| Patent document cited in search report | | | Publication date (day/month/year) | Patent family member(s) | Publication date (day/month/year) |
|---|---|---|---|---|---|
| WO | 2022/124170 | A1 | 16 June 2022 | (Family: none) | |
| JP | 2018-167000 | A | 01 November 2018 | US 2018/0289260 A1 entire text, all drawings | |
| WO | 2018/088338 | A1 | 17 May 2018 | (Family: none) | |

Form PCT/ISA/210 (patent family annex) (January 2015)

**REFERENCES CITED IN THE DESCRIPTION**

**Patent documents cited in the description**

- JP 2009543585 W
- US 7831106 B
- WO 2022124170 A
- US 8237835 B